(19) Europäisches Patentamt
European Patent Office
Office européen des brevets

(11) **EP 4 070 786 B1**

(12) **EUROPEAN PATENT SPECIFICATION**

(45) Date of publication and mention
of the grant of the patent:
**28.01.2026 Bulletin 2026/05**

(21) Application number: **20895953.6**

(22) Date of filing: **02.12.2020**

(51) International Patent Classification (IPC):
*A61K 9/00* (2006.01)     *A61K 9/19* (2006.01)
*A61K 9/51* (2006.01)     *A61K 47/42* (2017.01)
*A61K 47/44* (2017.01)     *A61K 31/015* (2006.01)
*A61P 35/00* (2006.01)     *A61K 45/06* (2006.01)

(52) Cooperative Patent Classification (CPC):
**A61P 35/00; A61K 9/0019; A61K 9/19;
A61K 9/5169; A61K 31/015; A61K 45/06;**
A61K 47/42; A61K 47/44

(86) International application number:
**PCT/CN2020/133470**

(87) International publication number:
**WO 2021/110073 (10.06.2021 Gazette 2021/23)**

(54) **PHARMACEUTICAL COMPOSITION CONTAINING ELEMENE, PREPARATION METHOD THEREFOR, AND USE THEREOF**

PHARMAZEUTISCHE ZUSAMMENSETZUNG MIT ELEMEN, HERSTELLUNGSVERFAHREN DAFÜR SOWIE VERWENDUNG DAVON

COMPOSITION PHARMACEUTIQUE CONTENANT DE L'ÉLÉMÈNE, SON PROCÉDÉ DE PRÉPARATION ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.12.2019 CN 201911217202**

(43) Date of publication of application:
**12.10.2022 Bulletin 2022/41**

(73) Proprietor: **SICHUAN HONGHE BIOTECHNOLOGY CO., LTD
Nanchong, Sichuan 637500 (CN)**

(72) Inventors:
• **GONG, Tao**
  **Chengdu, Sichuan 610036 (CN)**
• **KE, Xiao**
  **Chengdu, Sichuan 610036 (CN)**
• **ZHENG, Qiang**
  **Chengdu, Sichuan 610036 (CN)**
• **YE, Liang**
  **Chengdu, Sichuan 610036 (CN)**

(74) Representative: **Dehns
10 Old Bailey
London EC4M 7NG (GB)**

(56) References cited:
| | |
|---|---|
| **CN-A- 1 200 266** | **CN-A- 1 408 347** |
| **CN-A- 101 306 181** | **CN-A- 101 921 164** |
| **CN-A- 105 998 019** | **CN-A- 108 853 513** |
| **CN-A- 109 330 994** | **CN-A- 109 432 384** |
| **CN-A- 109 562 080** | **CN-A- 110 404 067** |
| **CN-C- 100 420 445** | **US-A1- 2004 192 641** |
| **US-A1- 2005 186 230** | **US-A1- 2005 186 230** |
| **US-A1- 2014 045 927** | |

• **LIU YANJU, LI XIAOFEI; FAN HANG; LU LINA: "Study on interaction between β-elemene and bovine serum albumin by fluorescence spectroscopy and molecular modeling", CHINA MEASUREMENT & TEST, vol. 41, no. 9, 1 September 2015 (2015-09-01), pages 51 - 55, XP055818737, DOI: 10.11857/ j.issn.1674-5124.2015.09.012**

- NIU XIAOYING, XIAOJING SUN, YANJU LIU: "Study on the Interaction Between β-elemene Glycine and Bovine Serum Albumin by Double Spectroscopy", ZHONG GUO YI XUE ZA ZHI = JOURNAL OF CHINESE MEDICINE, CN, vol. 34, no. 250, 1 March 2019 (2019-03-01), CN, pages 581 - 586, XP055818740, ISSN: 1674-8999, DOI: 10.16368/j.issn.1674-8999.2019.03.137

- ZHAN SHEN, WEN-BIN CHEN: "Transferrin-functionalized co-delivery microemulsion of β-elemene and celastrol for synergistic anti-colorectal cancer treatment", CHINESE TRADITIONAL AND HERBAL DRUGS, vol. 50, no. 2, 1 January 2019 (2019-01-01), pages 471 - 480, XP055818745, DOI: 10.7501/j.issn.0253-2670.2019.02.029

**Description**

FIELD OF THE INVENTION

The present invention relates to the field of pharmaceutical formulations, in particular, to a pharmaceutical composition comprising elemene and preparation methods and use thereof.

BACKGROUND OF THE INVENTION

Elemene is a volatile oily compound extracted from plants such as *Curcuma Wenyujin Y. H. Chen et C. ling* and *Cymbopoqon citratus ((DC.)) Stapt.* This compound appears as a pale yellow or yellow clear liquid with a pungent fennel smell. Currently, elemene is clinically used as a mixture containing α-elemene, β-elemene, δ-elemene and γ-elemene, which is widely used in malignant pleural effusion, lung cancer, gastrointestinal tumors and other superficial tumors. Since the elemene available in market and its formulations contain Cremophor EL and other excipients are highly irritating, most patients will suffer from serious phlebitis, fever, local pain, allergic reaction or mild digestive tract reaction, as well as pyrexia and other adverse reactions after the use of the drugs available in market. In the actual clinical medication process, due to the high incidence of phlebitis and higher recurrence rate thereof, the speed of intravenous infusion should be strictly controlled, resulting in a long infusion time.

Elemene is almost insoluble in water and has strong lipid solubility, especially in organic solvents such as petroleum ether, diethyl ether and the like. Its molecular formula is $C_{15}H_{24}$ and only consists of hydrocarbons. Due to its unique chemical structure and physicochemical properties, there are great difficulties and obstacles in its preparation of pharmaceutical formulations. In order to increase the solubility of elemene, the Cremophor EL and other excipients were added in drug prescription available in market during the preparation of elemene into injection, resulting in problems of serious hemolysis and great irritation. In addition, although the marketing of paclitaxel albumin nano-formulation provides a new form of pharmaceutical formulation for solving clinical side effects, due to the physicochemical properties of elemene, which is oily at room temperature, it is extremely challenging to formulate elemene into albumin formulations. Therefore, there is currently no corresponding research or development.

US 2005/186230 discloses injectable water-in-oil emulsions that comprise an elemene, a liquid oil, and a pharmaceutically acceptable aqueous phase.

SUMMARY OF THE INVENTION

The present application provides a pharmaceutical composition comprising elemene, an oil for injection and a protein carrier, which is able to effectively reduce one or more side effects of pharmaceutical compositions administered into the human body. The oil for injection is soybean oil and the protein carrier is human serum albumin. The weight ratio of the soybean oil to the elemene is about 0.35-3 and the weight ratio of the human serum albumin to the elemene is about 0.5-20.

The protein carrier used in the pharmaceutical composition is human serum albumin (HSA). Human serum albumin is a highly soluble globulin, Mr65K, composed of 585 amino acids. HSA is the most abundant protein in plasma and constitutes 70-80% of the colloidal osmotic pressure in human plasma.

The "oil for injection" used in the pharmaceutical composition is soybean oil.

In one or more embodiments of the present application, the pharmaceutical composition may be formulated into particles, wherein the particle size of the particles is less than 180 nm, for example, about 70-170 nm, preferably about 70-150 nm.

In one or more embodiments of the present application, the composition comprises nanoparticles of any shape (for instance, spherical or non-spherical shape), and in some embodiments, the average or intermediate diameter of the particles is no greater than about 180 nm. In some embodiments, the average or intermediate diameter of the particles is from about 50 nm to about 180 nm. In some embodiments, the average or intermediate diameter of the particles is from about 60 nm to about 180 nm. In some embodiments, the average or intermediate diameter of the particles is from about 70 nm to about 170 nm. In some embodiments, the average or intermediate diameter of the particles is from about 70 nm to about -170 nm. In some embodiments, the average or intermediate diameter of the particles is from about 70 to about 150 nm. In some embodiments, the average or intermediate diameter of the particles is about 60 nm, 70 nm, 80 nm, 90 nm, 100 nm, 110 nm, 120 nm, 130 nm, 140 nm, or 150 nm. In some implementations, the particles are sterile and filterable.

In one or more embodiments of the present application, the elemene in the pharmaceutical composition is selected from one or more of α-elemene, β-elemene, γ-elemene and δ-elemene. In one or more embodiments of the present application, the elemene in the pharmaceutical composition is β-elemene.

In one or more embodiments of the present application, the weight ratio of the protein carrier to the elemene in the pharmaceutical composition is 0.5-10.

In one or more embodiments of the present application, the pharmaceutical composition further contains a lyoprotectant. The lyoprotectant is selected from one or more of glucose, sucrose, maltose, lactose, mannose, trehalose, glycine and dextran, preferably trehalose or sucrose. In one or more embodiments, wherein the ratio of the weight of the lyoprotectant to the volume of the solution of the pharmaceutical composition is 3:100-20:100 g/mL, preferably 5:100-10:100 g/mL.

In one or more embodiments of the present application, the pharmaceutical composition further contains one or more of isoosmotic adjusting agent, antioxidant, preservative, and pH regulator. The isoosmotic adjusting agent is one or more of glycerol, sorbitol, mannitol or glucose; the pH regulator is one or more of sodium hydroxide, sodium citrate, citric acid, phosphoric acid, acetic acid or hydrochloric acid; the preservative is one or more of hydroxybenzene alkyl esters, benzoic acid, sodium benzoate, sorbic acid, chlorhexidine acetate, benzalkonium bromide; the antioxidant is one or more of sodium sulfite, sodium bisulfite, sodium metabisulfite, sodium thiosulfate, ascorbic acid, butylated hydroxyanisole, 2,6-di-tert-butylated hydroxytoluene, and vitamin E.

In one or more embodiments of the present application, there is also provided a pharmaceutical composition that no organic solvents other than the "oil for injection" is used during the preparation process thereof.

In one or more embodiments of the present application, the pharmaceutical composition may be formulated into particles, wherein the particle size of the particles is less than 180 nm, for example, about 70-150 nm.

In one or more embodiments of the present application, the weight ratio of the soybean oil to the elemene is about 0.5-3, the weight ratio of the human serum albumin to the elemene is about 0.5-2.5; or in the pharmaceutical composition, the weight ratio of the soybean oil to the elemene is about 0.35-1.5, and the weight ratio of the human serum albumin to the elemene is about 1.5-10.

In one or more embodiments of the present application, the weight ratio of soybean oil to elemene is about 0.5, and the weight ratio of human serum albumin to elemene is about 1.5.

In one or more embodiments of the present application, the weight ratio of soybean oil to elemene is about 0.75, and the weight ratio of human serum albumin to elemene is about 5.

In one or more embodiments of the present application, the weight ratio of soybean oil to elemene is about 1.5, and the weight ratio of human serum albumin to elemene is about 3-5.

In one or more embodiments of the present application, there is also provided a pharmaceutical composition that contains an organic solvent during the preparation process. Wherein the organic solvent is selected from one or more of chloroform, dichloromethane, tertiary butanol, isopropanol, ethyl acetate, ethanol, tetrahydrofuran, dioxane, acetonitrile, acetone, dimethyl sulfoxide, dimethylformamide, and methylpyrrolidone. Wherein the liposoluble organic solvent is a mixed solvent of dichloromethane and ethanol.

Wherein the volume ratio of dichloromethane to ethanol is 1:1-8, preferably 1:4.

The pharmaceutical composition may be formulated into particles, and the particle size of the particles is about 70-150 nm.

In the pharmaceutical composition, the weight ratio of the soybean oil to the elemene can be about 2.4, the weight ratio of the human serum albumin to the elemene can be about 3.

One or more embodiments of the present application provide a method for preparing the pharmaceutical composition of the present application, which comprises the following steps:

(1) mixing elemene and the oil for injection evenly, optionally adding an organic solvent, to obtain a solution of the elemene and the oil for injection; dissolving the protein carrier in water to obtain a protein carrier solution;
(2) mixing the two solutions obtained in step (1) to form an emulsion;
(3) homogenizing the emulsion in step (2) under a high pressure, and optionally evaporating the emulsion under a reduced pressure to remove the organic solvent, to obtain a nanoparticle solution.

One or more embodiments of the present application provide a method for preparing the pharmaceutical composition of the present application, which comprises the following steps:

(1) mixing elemene and the oil for injection evenly;
(2) taking the albumin and dissolving the albumin in water;
(3) mixing the both to form an emulsion;
(4) homogenizing the emulsion in step (3) under a high pressure to obtain a solution of albumin nanoparticles.

In one or more embodiments of the present application, in step (2), a shearing or ultrasonic method is used for mixing; and in step (3), a method of high-pressure homogenization or micro-jet homogenization is used for homogenization.

In one or more embodiments of the present application, step (3) may further comprise: flushing the pipeline of the homogenizer with an appropriate amount of trehalose aqueous solution to replace the homogeneous solution remaining in the homogenizer, and then flushing the pipeline of the homogenizer with an appropriate amount of ultrapure water to replace the residual trehalose aqueous solution, combining the trehalose aqueous solution with the homogeneous solution, mixing evenly, and then filtering.

In one or more embodiments of the present application, wherein the shearing may be performed by shearing with a mixer.

In one or more embodiments of the present application, wherein the speed of shearing with the mixer is 5000 rpm-10,000 rpm.

In one or more embodiments of the present application, wherein the shearing time of the mixer is 1 min-20 min, preferably for example 2 min-10 min or, more preferably 1 min-10 min.

In one or more embodiments of the present application, wherein the shearing is preferably performed multiple times with the mixer, preferably more than 2 times.

In one or more embodiments of the present application, preferably the first time is shearing at 5000 rpm for 1 min, and the second time is shearing at 10,000 rpm for 5 min.

In one or more embodiments of the present application, the pressure of the high-pressure homogenization is 200-1,600 bar (20-160 MPa), preferably for example 250-1,550 bar (25-155 MPa).

In one or more embodiments of the present application, wherein the time of the high-pressure homogenization is 1-10 min, for example, preferably 2-10 min, such as further preferably 5-8 min.

In one or more embodiments of the present application, wherein the high-pressure homogenization is preferably performed multiple times, preferably for example more than 2 times.

In one or more embodiments of the present application, the pressure of the micro-jet homogenization is 20,000 psi (138 MPa).

In one or more embodiments of the present application, wherein the time of the micro-jet homogenization is 30 min.

One or more embodiments of the present application further provide a method for preparing the pharmaceutical composition of the present application, which uses an organic solvent in the preparation process.

In one or more embodiments of the present application, the method for preparing the pharmaceutical composition of the present application comprises the following steps:

(1) dissolving elemene and the oil for injection in a liposoluble organic solvent;
(2) taking the albumin and dissolving the albumin in water;
(3) mixing the two solutions obtained in step (1) and (2) to form an emulsion;
(4) homogenizing the emulsion in step (3);
(5) evaporating the emulsion in step (4) under a reduced pressure to remove the organic solvent, to obtain a solution of albumin nanoparticles.

In one or more embodiments of the present application, the organic solvent in step (1) is selected from one or more of chloroform, dichloromethane, tertiary butanol, isopropanol, ethyl acetate, ethanol, tetrahydrofuran, dioxane, acetonitrile, acetone, dimethyl sulfoxide, dimethylformamide, methylpyrrolidone; for example, a mixed solvent of dichloromethane and ethanol, and the volume ratio of ethanol to dichloromethane in the mixed solvent is 1:1-8, for example 1:4.

In one or more embodiments of the present application, in the step (3), a shearing or ultrasonic method may be used for mixing; and in the step (4), a high-pressure or micro-jet method may be used for homogenization.

In one or more embodiments of the present application, wherein the shearing may be performed by shearing with a mixer.

In one or more embodiments of the present application, wherein the speed of shearing with the mixer is 5,000-10,000 rpm.

In one or more embodiments of the present application, wherein the shearing time of the mixer is 1 min-20 min, for example 2-10 min.

In one or more embodiments of the present application, wherein the shearing is preferably performed multiple times with the mixer, preferably more than 2 times.

In one or more embodiments of the present application, preferably the first time is shearing at 5,000 rpm for 1 min, and the second time is shearing at 10,000 rpm for 5 min.

In one or more embodiments of the present application, wherein the pressure of the high-pressure homogenization is 200-1,600 bar (20-160 MPa), for example 250-1,550 bar (25-155 MPa).

In one or more embodiments of the present application, wherein the time of high-pressure homogenization is 1-10 min, for example 2-10 min, such as further preferably 5-8 min.

In one or more embodiments of the present application, wherein the high-pressure homogenization is preferably performed multiple times, for example more than 2 times.

In one or more embodiments of the present application, the pressure of the micro-jet homogenization is 20,000 psi (138 MPa).

In one or more embodiments of the present application, wherein the time of the micro-jet homogenization is 30 min.

In one or more embodiments of the present application, the method for preparing the pharmaceutical composition of the present application further comprises the step of lyophilizing the obtained solution of albumin nanoparticles.

One or more embodiments of the present application further provide the pharmaceutical composition of the present application for use in the prevention or treatment of cancer.

In one or more embodiments of the present application, the cancer involved in the present application includes adrenocortical carcinoma, cryptogenic myeloid metaplasia, AIDS-related cancer, anal caner, appendiceal cancer, astrocytoma, basal cell carcinoma, cholangiocarcinoma, bladder cancer, bone cancer, glioma, ependymoma, oligodendroglioma, meningioma, craniopharyngioma, hemangioblastoma, medulloblastoma, neuroectodermal tumor, visual pathway and hypothalamic glioma and glioblastoma, bronchial adenoma, carcinoid tumor, central nervous system lymphoma, cervical cancer, colon cancer, colorectal cancer, chronic myeloproliferative disorder, endometrial cancer, ependymoma, Ewing's tumor family, eye cancer, gallbladder cancer, gastric cancer, gastrointestinal carcinoid tumor, gastrointestinal stromal tumor, germ cell carcinoma, gestational trophoblastic tumor, head and neck cancer, liver cancer, laryngeal cancer, leukemia, lip and oral cancer, lung cancer, lymphoma, medulloblastoma, melanoma, mesothelioma, metastatic neck squamous cell carcinoma, multiple endocrine neoplasia syndrome, myelodysplastic syndrome, myelodysplastic/ myeloproliferative disease, carcinoma of nasal cavity and sinuses, neuroblastoma, neuroendocrine cancer, oropharyngeal cancer, nasopharyngeal cancer, brain tumor, bone metastasis cancer, intestinal cancer, esophageal cancer, ovarian cancer, pancreatic cancer, skin cancer, parathyroid carcinoma, penile carcinoma, peritoneal cancer, pharyngeal carcinoma, pheochromocytoma, pineoblastoma and supratentorial primitive neuroectodermal tumor, pituitary tumor, pleuropulmonary blastoma, lymphoma, primary central nervous system lymphoma, pulmonary lymphangiomyomatosis, rectal cancer, kidney cancer, renal pelvis and ureter cancer, rhabdomyosarcoma, salivary gland cancer, small intestine cancer, squamous epithelial cell carcinoma, testicular cancer, throat cancer, thymoma and thymic carcinoma, thyroid cancer, urethral cancer, vaginal cancer.

In one or more embodiments of the present application, the cancer involved in the present application includes lung cancer, liver cancer, esophageal cancer, nasopharyngeal cancer, brain tumor, bone metastasis cancer, gastric cancer, intestinal cancer, uterine cancer, cervical cancer, germ cell carcinoma, endometrial cancer, gestational trophoblastic tumor, breast cancer, skin cancer, lymphoma, leukemia, malignant melanoma.

In one or more embodiments of the present application, the brain tumor includes glioma, brain stem glioma, cerebellar or cerebral astrocytoma, malignant glioma, ependymoma, oligodendroglioma, meningioma, craniopharyngioma, hemangioblastoma, medulloblastoma, visual pathway and hypothalamic glioma or glioblastoma; the cerebellar or cerebral astrocytoma is fibrous cell astrocytoma or diffuse astrocytoma or anaplastic (malignant) astrocytoma.

In one or more embodiments of the present application, the pharmaceutical composition of the present application may be used in combination with radiotherapy and chemotherapy for lung cancer, liver cancer, esophageal cancer, nasopharyngeal cancer, brain tumor, bone metastasis and other malignant tumors to enhance the curative effect, reduce the toxic and side effects of radiotherapy and chemotherapy, and may also be used for interventional, intracavitary chemotherapy and the treatment of cancerous hydrothorax and ascite.

In one or more embodiments of the present application, the compounds of the present application may be used in combination with other drugs such as anticancer drugs, wherein the anticancer drugs include temozolomide, paclitaxel, docetaxel, taxane, gemcitabine, anti-VEGF drugs, PD-1 antibody drugs; wherein, anti-VEGF drugs include AVASTIN, ranibizumab, aflibercept or conbercept; wherein, PD-1 antibody drugs include nivolumab, pembrolizumab, toripalimab, sintilimab, cemiplimab, atezolizumab, avelumab, or durvalumab.

One or more embodiments of the present application provide a pharmaceutical composition of the present application for use in a method for the treatment of human disease, comprising the administration of a therapeutically effective amount of the pharmaceutical composition. The pharmaceutical composition is administered by parenteral, by inhalation, intraperitoneal, intravesical, intramuscular, intravenous, intratracheal, subcutaneous, intraocular, intrathecal, transdermal, rectal or vaginal.

In one or more embodiments of the present application, the pharmaceutical composition of the present application may be formulated into a pharmaceutical formulation suitable for intravenous administration, a pharmaceutical formulation for parenteral administration, a pharmaceutical formulation for gastrointestinal administration, an aerosol, a pharmaceutical formulation for vaginal administration or other suitable pharmaceutical formulation, the pharmaceutical formulation is a solid formulation, a liquid formulation or a gas formulation.

In one or more embodiments of the present application, the liquid formulation of the present application may be a stable aqueous suspension reconstituted from a sterile lyophilized powder.

One or more embodiments of the present application provide a sealed container, which may be a unit-dose container or a multi-dose container, comprising the pharmaceutical composition of the present application. The sealed container may be a prefilled syringe. The pharmaceutical composition is a liquid composition or a dry composition. The pharmaceutical composition is lyophilized and sterile.

In one or more embodiments of the present application, the nano-formulation or nanoparticles described herein may be present in the form of a dry formulation (for instance, a lyophilized composition) or suspended in a biocompatible medium. The suitable biocompatible medium includes, but is not limited to, water, aqueous buffered medium, saline, buffered saline, optionally buffered amino acid solution, optionally buffered protein solution, optionally buffered sugar solution, optionally buffered vitamin solution, optionally buffered synthetic polymer solution, lipid-containing emulsion, and the like.

Unless expressly stated otherwise, an "individual" as used herein is a mammal including, but not limited to, a primate, human, bovine, equine, feline, canine, or rodent.

As used herein, "treatment" is a method of obtaining beneficial or desired outcomes, including clinical outcomes. For the purposes of the present invention, beneficial or desired clinical outcomes include, but are not limited to, any one or more of the following: reducing one or more symptoms resulting from the disease, reducing the severity of the disease, stabilizing the disease (for instance, preventing or delaying the exacerbation of the disease), preventing or delaying the spread (e.g. metastasis) of disease, preventing or delaying the occurrence or recurrence of disease, preventing or slowing the progression of the disease, improving the state of the disease, providing remission (whether partial or total) of the disease, reducing the dose of one or more other drugs necessary to treat a disease, delaying the progression of the disease, increasing quality of life, and/or prolonging survival. In some embodiments, the composition reduces the severity of one or more symptoms associated with cancer, as compared to corresponding symptoms in the same subject prior to treatment or compared to corresponding symptoms in other subjects who do not receiving the composition, by at least about any one of 10%, 20%, 30%, 40%, 50%, 60%, 70%, 80%, 90%, 95%, or 100%. "Treatment" also includes alleviation of the pathological consequences of cancer. The present invention contemplates any one or more of these therapeutic aspects.

As used herein, "pharmaceutically acceptable" or "pharmacologically compatible" means a substance that is not biological or otherwise undesirable may be incorporated into a pharmaceutical composition administered to a patient, for instance, without causing any significant undesired biological effect or without interacting in a deleterious manner with any other ingredient contained in the composition. The pharmaceutically acceptable carrier or excipient preferably meets the standards required for toxicology and manufacturing testing.

Reference herein to "about" a value or parameter includes (and describes) embodiments that refer to the value or parameter itself. For example, description referring to "about X" includes description of "X".

There are a variety of suitable formulations for the pharmaceutical composition of the present invention. The following formulations and methods are exemplary only and not limiting.

Formulations for oral administration may be made of (a) a liquid solution, for instance, an effective amount of the active ingredient dissolved in a diluent such as water, saline or orange juice, (b) capsules, sachets or tablets, each containing a predetermined amount of the active ingredient in solid or granular form, (c) a suspension in an appropriate liquid, and (d) a suitable emulsion. Tablet forms may include one or more of lactose, mannitol, corn starch, potato starch, microcrystalline cellulose, arabic gum, gelatin, colloidal silicon dioxide, croscarmellose sodium, talc, magnesium stearate, stearic acid and other excipients, colorants, diluents, buffers, wetting agents, preservatives, flavoring agents and pharmacologically compatible excipients. Lozenge forms may contain the active ingredient in spices, usually sucrose and arabic gum or tragacanth, and troches containing the active ingredient in an inert matrix, such as gelatin and glycerin, or sucrose and arabic gum, emulsions, gels, etc., in addition to the active ingredient, excipients known in the art are also contained.

Formulations for parenteral administration include an aqueous and nonaqueous, isotonic sterile injection solution, which may contain antioxidants, buffers, bacteriostatic agents, and solutes for rendering the formulation isotonic with the blood of the intended recipient, as well as an aqueous and non-aqueous sterile suspension which may include suspending agents, solubilizers, thickening agents, stabilizers and preservatives. The formulation may be presented in unit-dose or multi-dose sealed containers, such as ampoules and vials, and may be stored in a freeze-drying (lyophilizing) condition immediately before use, requiring only the addition of a sterile liquid excipient, such as water, for injection. Extemporaneous injection solutions and suspensions may be prepared from some kinds of the sterile powders, granules and tablets as described above.

Aerosols comprise the pharmaceutical composition of the present invention, the pharmaceutical composition includes an aqueous and nonaqueous, isotonic sterile solution which may contain antioxidants, buffers, bacteriostatic agents, and solutes, as well as an aqueous and non-aqueous sterile suspension which may include suspending agents, solubilizers, thickening agents, stabilizers and preservatives; the pharmaceutical composition may be prepared as an aerosol formulation alone or in combination with other suitable components for administration by inhalation. These aerosol formulations may be placed in pressurized acceptable propellants such as difluoromethane, propane, nitrogen, and the like. They may also be formulated as a non-pressure formulation of drugs, for example in a nebulizer or atomizer.

Other suitable formulations are also possible, for example suppositories may be prepared by using various bases such as emulsified bases or water-soluble bases. Formulations for vaginal administration may be presented as pessaries, tampons, creams, gels, pastes, foams or spray formulations, containing suitable carriers known in the art in addition to the active ingredient.

## BRIEF DESCRIPTION OF THE DRAWINGS

Figure 1 shows the hydratization diameter distribution of β-elemene albumin nanoparticles;
Figure 2 shows the potential diagram of β-elemene albumin nanoparticles;
Figure 3 shows the transmission electron microscope image of β-elemene albumin nanoparticles;
Figure 4 shows the stability investigation of particle size of different lyophilized samples after reconstitution;
Figure 5 shows the content stability investigation of different lyophilized samples after storage;
Figure 6 shows the survival of tumor-bearing mice in each group;
Figure 7 shows the results of the hemolytic experiment;
Figure 8 shows the tumor growth curve of the orthotopic tumor model in U87R1 mice;
Figure 9 shows the survival curve of the orthotopic tumor model in U87R1 mice.

## DETAILED DESCRIPTION OF THE INVENTION

The following embodiments are further illustrations of the invention and are not a limitation of the scope of the invention. The present invention is further described in detail below with reference to the examples, but those skilled in the art should understand that the present invention is not limited to these examples and the preparation methods as used.

The soybean oil involved in the present invention comes from soybean oil (SIO, injection grade, Lot19-C4520/Lot18C4816); the human serum albumin (HSA) involved in the present invention comes from human serum albumin (20%, Baxter, LotA1U133A) or (20%, Grifols, LotALAFD03042).

The conversion 1 bar = 0.1 Megapascal (MPa) applies throughout the following Test Examples and Examples.

The conversion 1 psi = 6894.76 Pascal (Pa) applies throughout the following Test Examples and Examples.

The commonly used detection methods of the present invention include particle size and particle size distribution, content determination, hemolysis experiment, etc., and the details are as follows:

### Particle size and particle size distribution

The particle size and the particle size distribution were determined with a particle size analyzer (MALVERN ZETASIZER NANO ZS90). 25 $\mu$L of sample was taken, 1 mL of ultrapure water was added to dilute it for detection, detection angle: 90°; dispersion medium: water; temperature: 25°C.

The particle size was the Z-average value (the average particle size of the sample) measured by the particle size analyzer, and the particle size distribution was the PDI value measured by the particle size analyzer.

### Content determination

It was determined by HPLC (General Rule 0512 of the Fourth Part of Chinese Pharmacopoeia 2015 Edition).

Chromatographic column was CHIRALPAK® IG (4.6×150 mm, 5 $\mu$m), with acetonitrile-water (55:45) as mobile phase, at a flow rate of 1.0 mL/min. The injection volume was 20 $\mu$L, the column temperature was 30°C, and the detection wavelength was 210 nm.

Preparation of reference substance solution (0.2 mg/mL) (external standard method): about 10 mg of β-elemene reference substance was precisely weighed into a 50 mL volumetric flask with a small amount of acetonitrile added, acetonitrile was added to be dissolved and diluted to volume scale, the mixture was shaken well to obtain the reference substance solution.

Preparation of test solution: the cap was removed from 1 bottle of this product and the product was precisely weighed ($m_1$), an appropriate amount of water was added (to make the ratio of API to water of 20 mg:1mL), precisely weighed ($m_2$) and reconstituted by shaking. About 500 mg of the reconstituted solution (about equivalent to API 10 mg) ($m_3$) was precisely weighed and placed in a 50 mL volumetric flask, about 1.5 mL of water was added first, and then acetonitrile was slowly added to 80% of the full range, sonicated for 10 min, cooled down, and acetonitrile was added again to dilute to volume scale, the mixture was shaken well, and filtered through a nylon filter with a pore size of 0.45 $\mu$m, to obtain the test solution. After the reconstituted solution was used, the vial was washed with water and dried, and the empty vial was precisely weighed ($m_4$).

20 $\mu$L was precisely measured, injected into a liquid chromatograph, and the chromatogram was record. The content of β-elemene ($C_{15}H_{24}$) in the test sample was calculated by the peak area according to the external standard method.

Calculation formula was:

$$\text{Content}(\%) = C_R \times \frac{A_X \times 50 \times (m_2 - m_4)}{A_R \times m_3 \times (m_1 - m_4)} \times 100\%$$

in the formula,

$A_X$: API peak area in the test solution
$A_R$: peak area of the reference substance
$C_R$: concentration of the reference substance

**Method for the determination of hemolysis value**

Preparation of blood cell suspension: a few milliliters of blood was taken from rabbits (male New Zealand rabbits, laboratory-suitable rearing for 1 week, body weight 2.5 kg-3 kg, blood was taken from the heart), and put in a beaker, the blood was stirred with a glass rod or bamboo stick to remove fibrinogen and get defibrinated blood. 0.9% sodium chloride solution was added in an amount of about 10 times, mixed evenly, centrifuged at 1,500 rpm/min for 15 min, the supernatant was discarded, and the precipitated red blood cells were washed 2-3 times with 0.9% sodium chloride solution according to the above method, until the supernatant was not red. The obtained red blood cells were prepared into 2% blood cell suspension with 0.9% sodium chloride solution for later use.

Sample preparation: according to the sample content, a proper amount was taken and diluted successively with normal saline to obtain 1 mg/mL sample (actual content).

Reaction observation: each drug was added in the following manner, and after mixing, the mixture was immediately placed in a water bath at 37°C for incubation, and the hemolysis was observed after 3 hours.

Drug group: 2.5 mL of 2% blood cell suspension, 2.2 mL of normal saline, and 0.3 mL of drug were successively added to a test tube.

Positive group: 2.5 mL of 2% blood cell suspension and 2.5 mL of distilled water were successively added to a test tube.

Negative group: 2.5 mL of 2% blood cell suspension and 2.5 mL of normal saline were successively added to a test tube.

Determination of OD value: 100 uL of the supernatant was pipetted into a 96-well plate, and the OD value was measured at a wavelength of 540 nm.

The calculation method of hemolysis value: (OD value of the sample - OD value of the negative group) / (OD value of the positive group - OD value of the negative group).

**Test Example 1**

The preparation of β-elemene albumin nanoparticles included the following steps: 1) 200 mg of elemene and 600 mg of soybean oil were weighted, and dissolved in 2 mL of dichloromethane to obtain an oil phase. 2) 650 mg of human serum albumin was dissolved in water for injection to obtain an aqueous solution of human serum albumin with a mass volume percentage of 3.6% (g/mL), that is, to obtain an aqueous phase. 3) Under the action of water bath ultrasound, the oil phase was added dropwise to the aqueous phase, and sonicated by a probe for 8 min (250 W) to produce a primary emulsion. 4) The obtained primary emulsion was transferred to a high-pressure homogenizer for high-pressure homogenization, the homogenization pressure was 1300 bar, and the homogenization was performed for 9 times. The organic solvent was removed from the homogenized system by rotary evaporation, and a 0.22 μm microporous membrane was used for filtration and sterilization to obtain an aqueous solution of elemene albumin nanoparticles, and the metered volume was adjusted to 10 mg/mL with water for injection. 5) 3% sucrose was added to the obtained elemene albumin nanoparticles, and lyophilized. The lyophilization parameters were: pre-freezing at -45°C for 5h; starting the vacuum pump and keeping the vacuum degree at about 10 Pa, temperature programming, drying at -40°C for 4h, drying at -30°C for 10h, and then drying at -20°C, -10°C, 0°C, 5°C and 10°C for 4h respectively. The lyophilized samples prepared above were reconstituted in pure water, and the particle size, potential and microscopic morphology of the nanoparticles were investigated by the following methods.

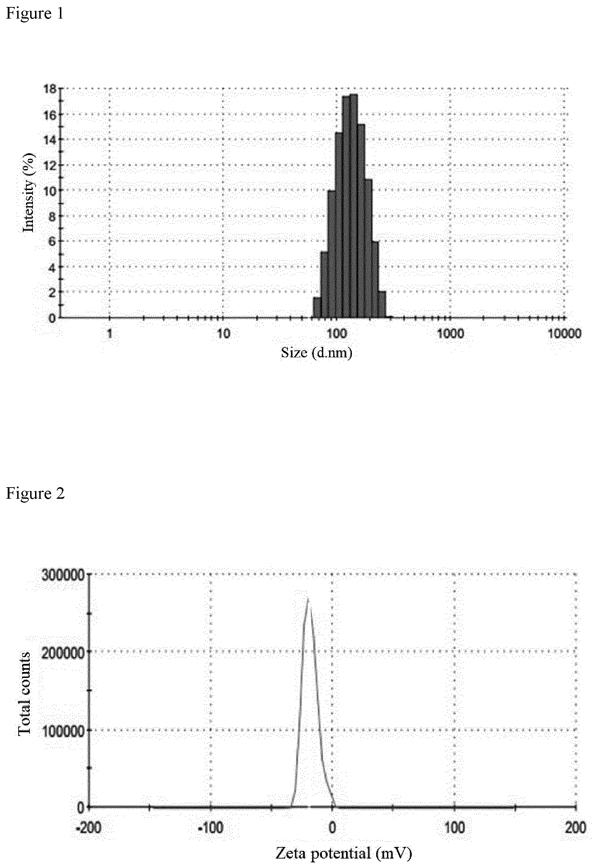

The Malvern particle size analyzer was preheated for 30 min before measurement, 50 μL of aqueous solution of the elemene albumin nanoparticles was taken, diluted to 1 mL with water, shaken slightly (to avoid bubbles), and placed in a Malvern Nano-ZS90 particle size dish or a potential dish to measure particle size and Zeta potential. The measurement temperature was 25°C, the equilibration time was 120 s, and the rest of the items were kept at the default values of the instrument, and the data was collected and processed by the Malvern V2.2 workstation. The particle size diagram and Zeta potential of the elemene albumin nanoparticles obtained were shown in Figure 1 and Figure 2. As shown in Figure 1 and 2, the particle size of the elemene albumin nanoparticles was about 110 nm, PDI was 0.127, and the potential was about -18 mV.

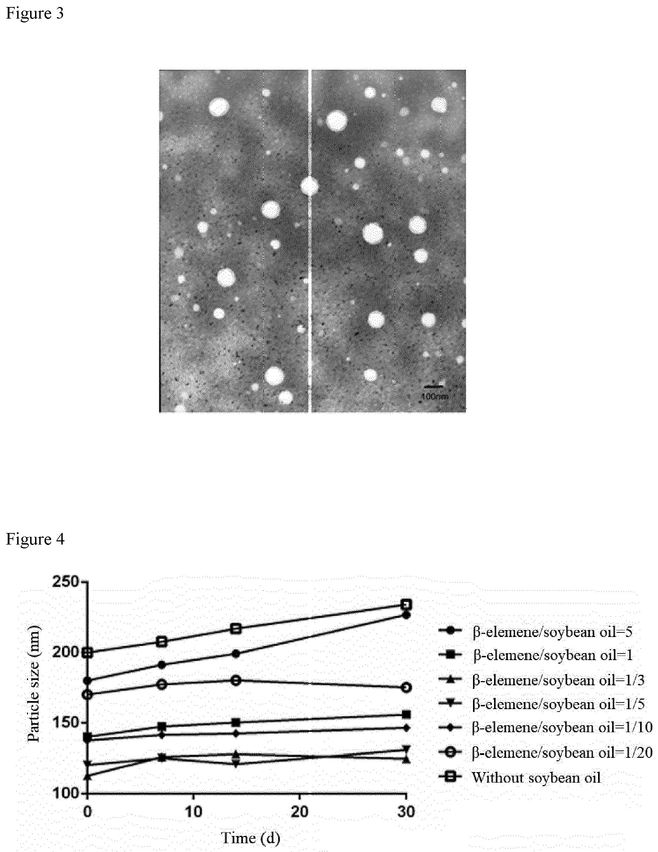

The aqueous solution of the elemene albumin nanoparticles was aspirated by a pipette and dropped on a copper mesh. After natural evaporation, 1% phosphotungstic acid solution was dropped on the copper mesh in the same way, and evaporated naturally, a few drops of pure water was aspirated to rinse the surface of the copper mesh carefully. After evaporation, the morphology was observed by transmission electron microscope as shown in Figure 3, the nanoparticles were spherical, regular in shape, relatively uniform in particle size, with a particle diameter of about 100 nm.

**Test Example 2**

Elemene albumin nanoparticles was prepared according to the method of Test Experiment 1, wherein the oil for injection was employed with the same amount as those of soybean oil, corn oil, camellia oil, and medium chain triglyceride (MCT) respectively to prepare elemene albumin nanoparticles using different oils for injection as raw materials. The particle size of the prepared nanoparticles was determined by a laser particle size analyzer. After lyophilization, the drug content of each group of formulations before and after lyophilization was determined respectively, and the drug loss rate during lyophilization was calculated.

The preparation of elemene albumin nanoparticles without soybean oil included the following steps: 1) 200 mg of elemene was weighed and dissolved in 2 mL of dichloromethane to obtain an oil phase. 2) 650 mg of human serum albumin was dissolved in water for injection to obtain an aqueous solution of human serum albumin with a mass volume percentage of 3.6% (g/mL), that is, to obtain an aqueous phase. 3) Under the action of water bath ultrasound, the oil phase was added dropwise to the aqueous phase, and sonicated by a probe for 8 min (250 W) to produce a primary emulsion. 4) The obtained primary emulsion was transferred to a high-pressure homogenizer for high-pressure homogenization, the homogenization pressure was 1300 bar, and the homogenization was performed for 9 times. The organic solvent was removed from the homogenized system by rotary evaporation, and a 0.22 $\mu$m microporous membrane was used for filtration and sterilization to obtain an aqueous solution of elemene albumin nanoparticles, and the metered volume was adjusted to 10 mg/mL with water for injection. 5) 3% sucrose was added to the obtained elemene albumin nanoparticles, and lyophilized according to the lyophilization parameters of Test Example 1. The drug content of a certain volume of the formulation before and after lyophilization was determined respectively, and the drug loss rate during lyophilization was calculated.

In addition, the groups of soybean oil, corn oil, camellia oil, and medium chain triglyceride only comprising no lyoprotectant of 3% sucrose were prepared according to the above methods, and the drug content of each certain volume of the formulations before and after lyophilization was determined respectively, and the drug loss rate during lyophilization was calculated. The results are shown in Table 1.

**Table 1: Drug loss rate of samples prepared with different oils for injection after lyophilization**

| Lyophilization loss rate | Soybean oil | Corn oil* | Camellia oil* | MCT* | None* |
|---|---|---|---|---|---|
| 3% sucrose contained | 7.1% | 10.4% | 9.2% | 11.8% | 31.4% |
| Sucrose free | 9.5% | 13.3% | 11.1% | 13.0% | 39.5% |

*Samples do not form part of the invention.

Experiments show that the lyophilization loss rates of albumin nanoparticles prepared by mixing oil for injection with elemene were reduced in different degrees compared with nanoparticles prepared without oil for injection.

**Test Example 3**

According to the method of Test Example 1, the amount of soybean oil was adjusted, the elemene albumin nanoparticles were prepared by feeding according to the mass ratio of $\beta$-elemene/soybean oil of 5:1, 1:1, 1:3, 1:5, 1:10, 1:20 and without oils for injection. Examples with a mass ratio of $\beta$-elemene/soybean oil of 5:1, 1:5, 1:10 and 1:20 do not form part of the invention.

The particle size of the prepared nanoparticles was determined by a laser particle size analyzer. After adding 3% sucrose and lyophilizing, the lyophilized samples were placed in a room temperature dryer. The samples were taken out periodically and reconstituted, and the changes of particle size and main drug content of the lyophilized samples within 30 days were determined. Specific detection parameters were as follows: instrument model: MALVERN ZETASIZER NANO ZS90, detection angle: 90°; detection mode: automatic; detection times: 3; detection type: nanoparticles; refractive index of the sample to be determined: 1.340; dispersion medium: water; temperature: 25°C; viscosity: 0.8872cp; refractive index of dispersion medium: 1.330; equilibration time: 120s; type of particle size dish: DTS0012.

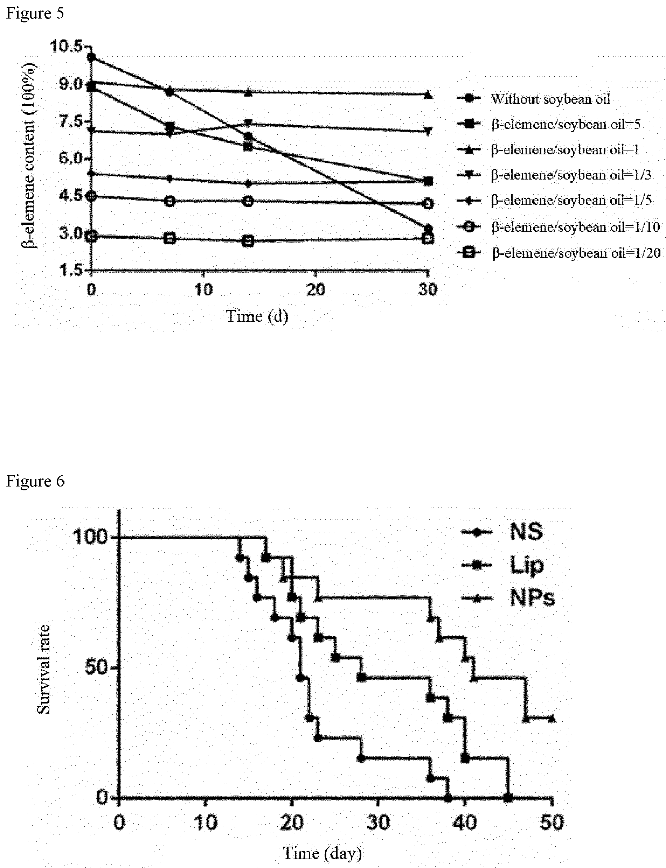

The results were shown in Figure 4 and Figure 5. The particle size of the formulation without soybean oil during the preparation process was more than 200 nm after lyophilization. With the prolongation of storage time, the particle size of the sample after reconstitution showed a trend of increasing gradually, and the drug content showed a trend of decreasing. Gradually increasing the proportion of soybean oil in the formulation, the particle size of the lyophilized samples after reconstitution was all less than 200 nm, the stability of particle size was better, and the stability of the main drug content was also gradually enhanced. Wherein, when the mass ratio of soybean oil/elemene was in the range of 1-10, the prepared lyophilized sample of nanoparticles had no aggregation phenomenon within 30 days at room temperature, the particle size was 100-150 nm, the PDI was less than 0.3, and the content of the main drug of β-elemene changed little. When the amount of elemene was 1 part and the amount of soybean oil was less than 1 part, the prepared nanoparticles did not show a good enough stability.

## Test Example 4

To investigate the therapeutic effect of the elemene albumin nanoparticles on mouse brain glioma, the experimental method was as follows.

Tumor modeling: the well-grown C6 cells in logarithmic phase were digested with trypsin, centrifuged, and the upper waste solution was discarded. The cells were washed with PBS, centrifuged, and the upper waste solution was discarded, and the cells were resuspended in PBS to make the final C6 cell density about $1 \times 10^6$ cells/5 $\mu$L. Healthy male Kunming mice (28-30 g) were anesthetized by intraperitoneal injection of an appropriate amount of 4% chloral hydrate. After shaving the hair on the top of the head, 75% ethanol was applied for disinfection, and a small incision was made along the sagittal midline of the head with sterile ophthalmic scissors, the tissue membrane was quickly removed with a cotton swab moistened with 10% hydrogen peroxide, the skull was separated, and the hydrogen peroxide was washed away with a cotton swab moistened with PBS. The head of the mouse was fixed on a brain stereotaxic device, located at a position 1.8 mm to the right and 0.6 mm to the back of the bregma, a small hole was drilled at the locating point with the needle of a 2 mL syringe, which should not be too deep to avoid bleeding. 5 $\mu$L of the above-mentioned C6 cells with a density of $1 \times 10^6$ cells/5 $\mu$L was aspirated with a micro-injection needle. The needle was inserted by 4 mm and withdrawn by 1 mm along the vertical direction of the small hole to leave a space for cells, and the cell suspension was slowly injected into the brain. The microneedle was taken out after remaining in the position of the small hole for 5 min. The wound was sutured with surgical thread, and finally the sutured surface was smeared with a cotton swab moistened with double antibody.

On the 10th day after tumor inoculation, the tumor-bearing mice were divided into normal saline group (NS), commercially available elemene liposomes (Lip, Dalian Jingang, approval number: Guoyaozhunzi H10960114), elemene albumin nanoparticles (NPs) prepared in Test Example 1, with 12 mice in each group. The dosage of administration was 30 mg/kg, once every 2 days, for a total of 4 times. The survival time of each mouse was recorded, and the survival curve was made.

The investigation results of survival were shown in figure 6. The results of the experiment showed that the C6 glioma-bearing mice administered with the elemene albumin nanoparticles exhibited longer survival. The average survival of the normal saline group was 21 days, the average survival of the elemene liposome group was 28 days, and the average survival of the elemene albumin nanoparticles group was 41 days. Therefore, the elemene albumin nanoparticles prepared in the experiment have an effect of prolonging the survival of C6-bearing glioma mice.

## Test Example 5

Hemolysis experiment:

New Zealand rabbits (male, laboratory-suitable housed for 1 week, body weight 2.5 kg-3 kg) were divided into two groups of commercially available elemene injection (Ailineng, CSPC Yuanda Pharmaceutical Co., Ltd., batch number 17050302) and elemene albumin nanoparticles (NPs) prepared in Test Example 1, with 2 mice in each group, the dosage of administration was 50 mg/kg. The two formulations were respectively prepared into medicinal solutions having a concentration of 6 mg/mL with glucose solution, and then the rabbits were administered by intravenous infusion.

Within 10-15 minutes after administration, about 1 mL of venous blood was drawn from the ear vein and placed in a centrifuge tube coated with heparin sodium, centrifuged at 3500 r/min for 15 minutes, and the supernatant was taken to observe the color.

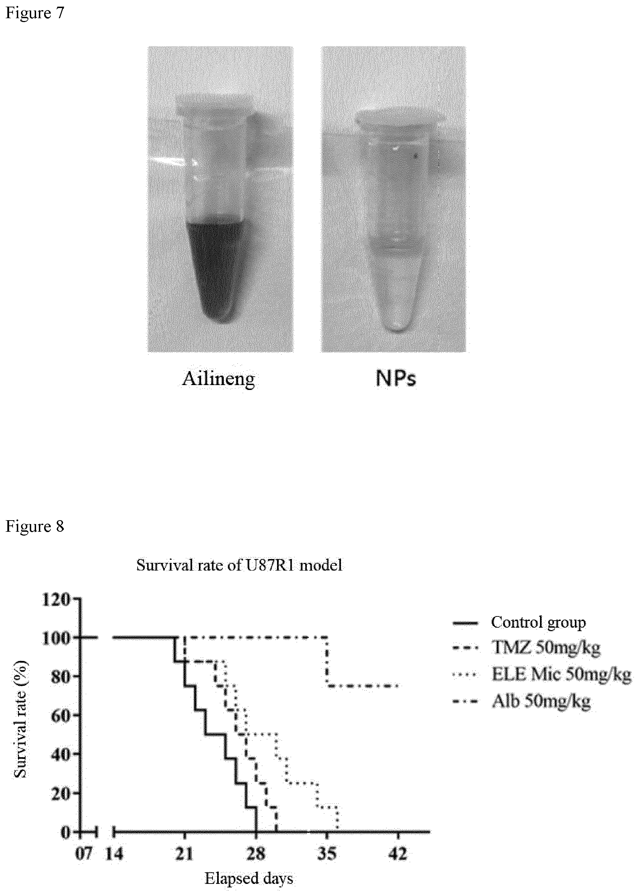

The color of the obtained plasma was shown in figure 7. The result showed that the New Zealand rabbits administered with the elemene albumin nanoparticles did not show hemolysis, demonstrating little irritation and high safety.

## Test Example 6

### 1.1 Preparation of experimental samples

**Sample 1**

3.33 g of β-elemene and 8 g of soybean oil were weighed and mixed to obtain an oil phase. 50 mL (10g) of 20% human serum albumin was measured, diluted evenly by adding an appropriate amount of ultrapure water, and the metered volume was adjusted to 333 mL to obtain an aqueous solution of human serum albumin with a mass volume percentage of 3% (g/mL), namely an aqueous phase. The aqueous phase was poured into the oil phase, and the mixer (FLUKO, FA30/30F) was used for shearing at 10,000 rpm for 10 min to form a primary emulsion. The obtained primary emulsion was transferred to a micro-jet homogenizer (Microfluidizer, M-110-EH) for homogenization, at a homogenization pressure of 20,000 psi for 30 min. The obtained emulsion was filtered by 0.45 μm and 0.22 μm PVDF plate filters, and the metered volume was adjusted, and 3% (g/mL) of sucrose was added as a lyoprotectant. The homogeneous solution added with the lyoprotectant was lyophilized, and after lyophilization, the lyophilized powder was reconstituted with sterilized ultrapure water. Particle size was 175.97 nm detected by a particle size analyzer, PDI was 0.188.

**Sample 2**

3.33 g of β-elemene and 8 g of soybean oil were weighed, dissolved in an organic solvent, and mixed to obtain an oil phase. The organic solvent was a mixed solvent of 26.67 mL dichloromethane and 6.67 mL anhydrous ethanol. 50 mL (10 g) of 20% human serum albumin was measured, diluted evenly by adding an appropriate amount of ultrapure water, and the metered volume was adjusted to 333 mL to obtain an aqueous solution of human serum albumin with a mass volume percentage of 3% (g/mL), namely an aqueous phase. The aqueous phase solution was poured into the oil phase, and the mixer (FLUKO, FA30/30F) was used for shearing at 10,000 rpm for 10 min to form a primary emulsion. The obtained primary emulsion was transferred to a micro-jet homogenizer (Microfluidizer, M-110-EH) for high-pressure homogenization, at a homogenization pressure of 20,000 psi for 30 min. After homogenization, the organic solvent was removed by a thin film evaporator (Shanghai DEA) from the organic solvent-containing emulsion, and the parameters were: temperature 30°C, vacuum degree 4000 pa, liquid feeding speed 100 rpm, scraper speed 50 rpm. The obtained emulsion was filtered by 0.45 μm and 0.22 μm PVDF plate filters, and the metered volume was adjusted, and 3% (g/mL) of sucrose was added as a lyoprotectant. The homogeneous solution added with the lyoprotectant was lyophilized, and after lyophilization, the lyophilized powder was reconstituted with sterilized ultrapure water. Particle size was 78.34 nm detected by a particle size analyzer, PDI was 0.207.

**Sample 3 (Elemene injection (trade name: Ailineng)):** CSPC Yuanda (Dalian) Pharmaceutical Co., Ltd., batch number: 19061502; particle size was 14.74 nm detected by a particle size analyzer, PDI: 0.035.

### 1.2 PK and mouse brain tissue distribution of elemene albumin nanoparticles

Kunming mice (male, SPF grade, body weight 20-25 g) were used for the experiment. The dosage of administration was 50 mg/kg, administered through the tail vein. Brain tissue samples were collected at different time periods and stored at -20°C in time for the detection of drug content in brain tissue. There were a total of 45 mice for three samples, 3 Kunming mice for each dosage form at each time point. The eyeballs were removed for blood collection at 5min, 15min, 30min, 1h, and 2h after administration, and brain tissues were collected to detect drug content in brain tissues. GC-MS was used to quantitatively analyze β-elemene in biological samples of plasma and brain tissue of the Kunming mice. The results of the study on the half-life in brain tissue and the brain tissue exposure are shown in Table 2.

**Table 2 Results of studies on half-life and brain tissue exposure**

|  | Sample 1 | Sample 2 | Sample 3* |
|---|---|---|---|
| Particle size (nm) | 175.97 | 78.34 | 14.7 |
| Half-life in brain tissue (min) | 41.8 | 57.5 | 33.6 |
| Brain drug exposure (mg/L*min) | 2808.7 | 3008.6 | 2812.7 |
| *Sample 3 does not form part of the invention. | | | |

**Test Example 7**

**Preparation and stability investigation of sample 4**

1 g of β-elemene and 3 g of soybean oil were weighed and mixed to obtain an oil phase. 15 mL (3 g) of 20% human serum albumin was measured, diluted evenly by adding 85 mL of ultrapure water, to obtain an aqueous solution of human serum albumin with a mass volume percentage of 3% (g/mL), namely an aqueous phase. The aqueous phase solution was poured into the oil phase, and the mixer was used for shearing at 5000 rpm for 1 min, and at 10,000 rpm for 5 min, to form a primary emulsion. The obtained primary emulsion was transferred to a high-pressure homogenizer for high-pressure homogenization at a homogenization pressure of 250-300 bar for 2 min, and at 1350-1400 bar for 9 min. After homogenization, the total volume of the homogeneous solution was measured with a measuring cylinder, which was about 50 mL, 5 g trehalose was added, shaken well to be dissolved, and filtered with a 0.45μm microporous membrane to obtain a solution of elemene albumin nanoparticles. The particle size was 141.0 nm detected by a particle size analyzer, PDI was 0.162. The homogeneous solution added with trehalose was lyophilized, and after lyophilization, the lyophilized powder was reconstituted with sterilized ultrapure water. The particle size was 144.07 nm detected by a particle size analyzer, PDI was 0.123. The loss rate of elemene content before and after lyophilization was 1.27%.

**Preparation and stability investigation of sample 5**

4 g of β-elemene and 2 g of soybean oil were weighed and mixed to obtain an oil phase. 30 mL (6 g) of 20% human serum albumin was measured, diluted evenly by adding 170 mL of ultrapure water, to obtain an aqueous solution of human serum albumin with a mass volume percentage of 3% (g/mL), namely an aqueous phase. The aqueous phase solution was poured into the oil phase, and the mixer was used for shearing at 5000 rpm for 1 min, and at 10,000 rpm for 10 min, to form a primary emulsion. The obtained primary emulsion was transferred to a high-pressure homogenizer for high-pressure homogenization at a homogenization pressure of 250-300 bar for 2 min, and at 1350-1400bar for 24 min. After homogenization, 150 mL of homogeneous solution was measured with a measuring cylinder, 15 g trehalose was added, shaken well to be dissolved, and filtered with a 0.45μm microporous membrane to obtain a solution of elemene albumin nanoparticles. The particle size was 132.4 nm detected by particle size analyzer, PDI was 0.133. The homogeneous solution added with trehalose was lyophilized, and after lyophilization, the lyophilized powder was reconstituted with sterilized ultrapure water. The particle size was 123.68 nm detected by a particle size analyzer, PDI was 0.144. The loss rate of elemene content after lyophilization was 1.58%.

**Preparation and stability investigation of sample 6**

Sample 6 does not form part of the invention.
1 g of β-elemene and 2 g of egg yolk lecithin were weighed, 50 mL of ultrapure water was added, and the mixer was used for shearing at 5000 rpm for 1 min, and shearing at 10,000 rpm for 5 min. Then the lecithin adhered to the bottle wall was scraped into the solution, and sheared at 15,000 rpm for 3 min again until no obvious undispersed lecithin was found, and a primary emulsion was obtained. The primary emulsion was transferred to a high-pressure homogenizer for high-pressure homogenization at a homogenization pressure of 250-300 bar for 2 min, and at 1300-1350 bar for 12 min. After homogenization, the homogeneous solution was filtered with a 0.45 μm filter membrane, and 50 mL of 2% HSA solution (containing 1 g of HSA) was added to the filtered homogeneous solution, and homogenized at 280 bar for 1 cycle; and the homogeneous solution was discharged, 40 mL was measured with a measuring cylinder, 4 g trehalose was added, shaken well to be dissolved, and filtered with a 0.45 μm microporous membrane to obtain a solution of albumin nanoparticles. The particle size was 150.86 nm detected by a particle size analyzer, PDI was 0.114. The homogeneous solution added with trehalose was lyophilized, and after lyophilization, the lyophilized powder was reconstituted with sterilized ultrapure water. The particle size was 181.56 nm detected by a particle size analyzer, PDI was 0.251. The loss rate of elemene content after lyophilization was 16.47%.

**Preparation and stability investigation of sample 7**

Sample 7 does not form part of the invention.
1 g of β-elemene, 2 g of egg yolk lecithin and 0.5 g of TPGS were weighed, 50 mL of ultrapure water was added, and the mixer was used for shearing at 5000 rpm for 1 min, and shearing at 10,000 rpm for 5 min. Then the lecithin adhered to the bottle wall was scraped into the solution, and sheared at 15,000 rpm for 3 min again until no obvious undispersed lecithin was found, and a primary emulsion was obtained. The primary emulsion was transferred to a high-pressure homogenizer for high-pressure homogenization at a homogenization pressure of 250-300 bar for 2 min, and at 1300-1350 bar for 12 min. After homogenization, the solution was filtered with a 0.22 μm filter membrane, and 50 mL of 2% HSA solution (containing 1 g of HSA) was added to the filtered homogeneous solution, homogenized at 140 bar for 1 cycle; and the homogeneous solution was discharged, 40 mL was measured with a measuring cylinder, 4 g of trehalose was added, shaken well to be dissolved, and filtered with a 0.45 μm microporous membrane to obtain a solution of albumin nanoparticles. The particle size was 93.11 nm detected by a particle size analyzer, PDI was 0.131. The homogeneous solution added with trehalose was lyophilized, and after lyophilization, the lyophilized powder was reconstituted with sterilized ultrapure water. The particle size was 811.70 nm detected by a particle size analyzer, PDI was 0.313. The loss rate of elemene content after lyophilization was 82.64%.

In the following, Examples 3-5, 7 and 9-18 do not form part of the invention. Some of the samples in Example 25 do not form part of the invention and are marked accordingly.

**Example 1**

200 mg of β-elemene and 600 mg of soybean oil were weighed, dissolved in 2 mL of an organic solvent, and mixed by vortex to obtain an oil phase. The organic solvent is dichloromethane, or ethyl acetate or a mixture of dichloromethane and ethanol. 650 mg of human serum albumin was taken and dissolved in 18 mL of water for injection to obtain an aqueous solution of human serum albumin with a mass volume percentage of 3.6% (g/mL), that is, to obtain an aqueous phase. Under the action of water bath ultrasound, the oil phase was added dropwise to the aqueous phase, and sonicated by a probe for 8 min (250 W) to produce a primary emulsion. The obtained primary emulsion was transferred to a high-pressure homogenizer for high-pressure homogenization, the homogenization pressure was 1000 bar, and the homogenization times was 16 times. The organic solvent was removed by rotary evaporation after homogenization, and a 0.22 μm microporous membrane was used for filtration and sterilization to obtain an aqueous solution of elemene albumin nanoparticles.

**Table 3 Investigation results of single factor for organic solvents**

| Number | Organic solvent | Particle size (d.nm) | PDI |
|---|---|---|---|
| 1 | Without organic solvent | 142.1 | 0.094 |
| 2 | Ethyl acetate | 122.0 | 0.164 |
| 3 | Dichloromethane | 108.8 | 0.129 |
| 4 | Dichloromethane/ethanol (4:1) | 98.5 | 0.130 |

The experimental results showed that, no matter whether β-elemene and soybean oil were dissolved in organic solvents or not during the preparation process, β-elemene albumin formulations with suitable particle size were prepared. Among various organic solvents, the nanoparticles prepared by dichloromethane or a mixed solvent of dichloromethane and ethanol had smaller particle size.

**Example 2**

200 mg of β-elemene and 600 mg of soybean oil were weighed, dissolved in a mixed solvent of dichloromethane and ethanol (dichloromethane:ethanol=4:1, 2 mL), and mixed by vortex to obtain an oil phase with a volume of 2 mL. 650 mg of human serum albumin was taken and dissolved in water for injection to obtain an aqueous solution of human serum albumin with a mass volume percentage of 3.6% (g/mL), and then 100 mg of polyethylene glycol-12-hydroxystearate was added, to obtain an aqueous phase. Under the action of water bath ultrasound, the oil phase was added dropwise to the aqueous phase, and sonicated by a probe for 8 min (250 W) to produce a primary emulsion. The obtained primary emulsion was transferred to a high-pressure homogenizer for high-pressure homogenization, the homogenization pressure was 1000 bar, and the homogenization times was 16 times. After homogenization, the organic solvent was removed by rotary evaporation, and a 0.22 μm microporous membrane was used for filtration and sterilization to obtain an aqueous solution of elemene albumin nanoparticles. The measured particle size was 116.4 nm, PDI was 0.118.

**Example 3**

200 mg of β-elemene and 400 mg of medium and long chain fatty glyceride were weighed, dissolved in a mixed solvent of dichloromethane and ethanol (dichloromethane:ethanol=4:1, 2 mL), and mixed by vortex, to obtain an oil phase with a volume of 2 mL. 900 mg of human serum albumin was taken and dissolved in 18 mL of water for injection to obtain an aqueous solution of human serum albumin with 5.0% of mass volume percentage (g/mL), that is, to obtain an aqueous phase. Under the action of water bath ultrasound, the oil phase was added dropwise to the aqueous phase, and sonicated by a probe for 8 min (250 W) to produce a primary emulsion. The obtained primary emulsion was transferred to a high-pressure homogenizer for high-pressure homogenization, the homogenization pressure was 1000 bar, and the homogenization times was 16 times. After homogenization, the organic solvent was removed by rotary evaporation, and a 0.22 $\mu$m microporous membrane was used for filtration and sterilization to obtain an aqueous solution of elemene albumin nanoparticles. The measured particle size was 105.3 nm, PDI was 0.116.

**Example 4**

800 mg of $\beta$-elemene and 2400 mg of sesame oil were weighed, dissolved in a mixed solvent of dichloromethane and ethanol (dichloromethane:ethanol=4:1, 10 mL), and mixed by vortex to obtain an oil phase. 4000 mg of human serum albumin was taken and dissolved in 100 mL of water for injection to obtain an aqueous solution of human serum albumin with 4% of mass volume percentage (g/mL), that is, to obtain an aqueous phase. The oil phase was added dropwise to the aqueous phase, and a tissue masher was used at 10,000 rpm for shearing for 10 min to prepare a primary emulsion. The obtained primary emulsion was transferred to a high-pressure homogenizer for high-pressure homogenization, the homogenization pressure was 1000 bar, and the homogenization times was 20 times. After homogenization, the organic solvent was removed by rotary evaporation, and a 0.22 $\mu$m microporous membrane was used for filtration and sterilization to obtain an aqueous solution of elemene albumin nanoparticles. The measured particle size was 121.3 nm, PDI was 0.133.

**Example 5**

800 mg of $\beta$-elemene and 2400 mg of olive oil were weighed, successively added to a dichloromethane/ethanol mixed solvent (dichloromethane:ethanol=4:1, 5 mL), and mixed by vortex to obtain an oil phase. 4000 mg of human serum albumin was taken and dissolved in water for injection to obtain an aqueous solution of human serum albumin with a mass volume percentage of 4% (g/mL), to obtain an aqueous phase. The oil phase was added dropwise to the aqueous phase, and a tissue masher was used at 12000 rpm for shearing for 10 min to prepare a primary emulsion. The obtained primary emulsion was transferred to a high-pressure homogenizer for high-pressure homogenization, the homogenization pressure was 1400 bar, and the homogenization times was 20 times. The organic solvent was removed from the homogenized system by rotary evaporation. The rotary-evaporated medicinal solution was filtered and sterilized via a 0.22 $\mu$m microporous membrane to obtain an aqueous solution of elemene albumin nanoparticles. 3% sucrose was added and lyophilized, capped after lyophilization, and stored at 4°C. The lyophilized samples were taken and reconstituted in water for injection, and the measured particle size was 112.6 nm, PDI was 0.101.

**Example 6**

200 mg of $\beta$-elemene and 600 mg of soybean oil were weighed, dissolved in a mixed solvent of dichloromethane and ethanol (dichloromethane:ethanol=4:1, 10 mL), and mixed by vortex to obtain an oil phase. 4000 mg of human serum albumin was dissolved in water for injection to obtain an aqueous solution of human serum albumin with 10% of mass volume percentage (g/mL), that is, to obtain an aqueous phase. Under the action of water bath ultrasound, the oil phase was added dropwise to the aqueous phase, and sonicated by a probe for 8 min (250 W) to produce a primary emulsion. The obtained primary emulsion was transferred to a high-pressure homogenizer for high-pressure homogenization, the homogenization pressure was 1000 bar, and the homogenization times was 16 times. After homogenization, the organic solvent was removed by rotary evaporation, and a 0.22 $\mu$m microporous membrane was used for filtration and sterilization to obtain an aqueous solution of elemene albumin nanoparticles. The measured particle size was 140.3 nm, PDI was 0.164.

**Example 7**

200 mg of $\beta$-elemene and 1000 mg of soybean oil were weighed, dissolved in a mixed solvent of dichloromethane and ethanol (dichloromethane:ethanol=4:1, 5 mL), and mixed by vortex to obtain an oil phase. 650 mg of human serum albumin was taken and dissolved in water for injection to obtain an aqueous solution of human serum albumin with 3.6% of mass volume percentage (g/mL), that is, to obtain an aqueous phase. Under the action of water bath ultrasound, the oil phase was added dropwise to the aqueous phase, and sonicated by a probe for 8 min (250 W) to produce a primary emulsion. The obtained primary emulsion was transferred to a high-pressure homogenizer for high-pressure homogenization, the homogenization pressure was 1000 bar, and the homogenization times was 16 times. After homogenization, the organic solvent was removed by rotary evaporation, and a 0.22 $\mu$m microporous membrane was used for filtration and sterilization to obtain an aqueous solution of elemene albumin nanoparticles. The measured particle size was 125.3 nm, PDI was 0.148.

### Example 8

800 mg of β-elemene and 2400 mg of soybean oil were weighed, dissolved in a mixed solvent of dichloromethane and ethanol (dichloromethane:ethanol=4:1, 10 mL), and mixed by vortex to obtain an oil phase. 4000 mg of human serum albumin was dissolved in water for injection to obtain an aqueous solution of human serum albumin with 4% of mass volume percentage (g/mL), that is, to obtain an aqueous phase. The oil phase was added dropwise to the aqueous phase, and a tissue masher was used at 10000 rpm for shearing for 10 min to prepare a primary emulsion. The obtained primary emulsion was transferred to a high-pressure homogenizer for high-pressure homogenization, the homogenization pressure was 1000 bar, and the homogenization times was 20 times. After homogenization, the organic solvent was removed by rotary evaporation, and a 0.22 μm microporous membrane was used for filtration and sterilization to obtain an aqueous solution of elemene albumin nanoparticles. The measured particle size was 119.3 nm, PDI was 0.143. The prepared nanoparticle solution was taken and configured to a drug concentration of 10 mg/mL, an appropriate amount of lyoprotectant was added, and the amount of the lyoprotectant was 3% (g/mL), and lyophilized. After lyophilization, water for injection was added to reconstitute, and the particle size was determined. The results are shown in the following table.

**Table 4 Particle size of elemene albumin nanoparticles with different lyoprotectants after lyophilization**

| Lyoprotectant | None | Glucose | Sucrose | Trehalose | Mannitol | Lactose | Maltose |
|---|---|---|---|---|---|---|---|
| Particle size (nm) | 187.4 | 160.7 | 153.5 | 167.4 | 172.1 | 166.3 | 164.5 |
| PDI | 0.233 | 0.165 | 0.125 | 0.167 | 0.133 | 0.178 | 0.196 |

The experimental results showed that, during the lyophilization process of elemene albumin nanoparticles, various lyoprotectants were added to obtain lyophilized powders with loose structure and uniform texture, which could be reconstituted by adding water, and the particle size after reconstitution did not exceed 200 nm. Wherein, the nanoparticles obtained by reconstituting the sample by lyophilizing with sucrose as the lyoprotectant had the smallest particle size.

### Example 9

500 mg of β-elemene and 1200 mg of medium and long chain fatty glyceride were weighed, dissolved in a mixed solvent of dichloromethane and ethanol (dichloromethane:ethanol=4:1, 5 mL), and mixed by vortex, to obtain an oil phase. 1500 mg of human serum albumin was taken and dissolved in 50 mL of water for injection to obtain an aqueous solution of human serum albumin with 3% of mass volume percentage (g/mL), to obtain an aqueous phase. The oil phase was added dropwise to the aqueous phase, and sonicated by a probe for 8 min (250 W) to produce a primary emulsion. The obtained primary emulsion was transferred to a high-pressure homogenizer for high-pressure homogenization, the homogenization pressure was 1400 bar, and the homogenization times was 10 times. After homogenization, the organic solvent was removed by rotary evaporation, and a 0.22 μm microporous membrane was used for filtration and sterilization, the filtrate was diluted and configured to a drug concentration of 15 mg/mL, 3% sucrose was added, lyophilized, capped, and stored at 4°C. The lyophilized sample was taken and reconstituted in water for injection, and the particle size was determined. The measured particle size was 103.5 nm, PDI was 0.123.

### Example 10

800 mg of β-elemene and 2400 mg of sesame oil were weighed, successively added to a dichloromethane/isopropanol mixed solvent (dichloromethane:isopropanol=4:1, 5 mL), and mixed by vortex to obtain an oil phase. 4000 mg of human serum albumin was dissolved in water for injection to obtain an aqueous solution of human serum albumin with a mass volume percentage of 4% (g/mL), to obtain an aqueous phase. The oil phase was added dropwise to the aqueous phase, and a tissue masher was used at 10,000 rpm for shearing for 10 min to prepare a primary emulsion. The obtained primary emulsion was transferred to a high-pressure homogenizer for high-pressure homogenization, the homogenization pressure was 1000 bar, and the homogenization times was 20 times. The organic solvent was removed from the homogenized system by rotary evaporation. The rotary-evaporated medicinal solution was filtered and sterilized via a 0.22 μm microporous membrane to obtain an aqueous solution of elemene albumin nanoparticles. The aqueous solution was diluted and configured to a drug concentration of 10 mg/mL, and 3% sucrose was added for lyophilization, capped after lyophilization, and stored at 4°C. The lyophilized sample was taken and reconstituted in water for injection, and the measured particle size was 115.2 nm, PDI was 0.102.

**Example 11**

800 mg of β-elemene and 2400 mg of olive oil were weighed, successively added to a dichloromethane/tertiary butanol mixed solvent (dichloromethane:tertiary butanol=4:1, 5 mL), and mixed by vortex to obtain an oil phase. 4000 mg of human serum albumin was dissolved in water for injection to obtain an aqueous solution of human serum albumin with a mass volume percentage of 4% (g/mL), to obtain an aqueous phase. The oil phase was added dropwise to the aqueous phase, and a tissue masher was used at 10,000 rpm for shearing for 10 min to prepare a primary emulsion. The obtained primary emulsion was transferred to a high-pressure homogenizer for high-pressure homogenization, the homogenization pressure was 1000 bar, and the homogenization times was 20 times. The organic solvent was removed from the homogenized system by rotary evaporation. The rotary-evaporated medicinal solution was filtered and sterilized via a 0.22 μm microporous membrane to obtain an aqueous solution of elemene albumin nanoparticles. The aqueous solution was diluted and configured to a drug concentration of 10 mg/mL, and 3% sucrose was added for lyophilization, capped after lyophilization, and stored at 4°C. The lyophilized sample was taken and reconstituted in water for injection, and the measured particle size was 109.6 nm, PDI was 0.113.

**Example 12**

800 mg of β-elemene and 2400 mg of sesame oil were weighed, successively added to a dichloromethane/tetrahydrofuran mixed solvent (dichloromethane:tetrahydrofuran=4:1, 5 mL), and mixed by vortex to obtain an oil phase with a volume of 5 mL. 4000 mg of human serum albumin was taken and dissolved in water for injection to obtain an aqueous solution of human serum albumin with a mass volume percentage of 4% (g/mL), to obtain an aqueous phase. The oil phase was added dropwise to the aqueous phase, and a tissue masher was used at 10,000 rpm for shearing for 10 min to prepare a primary emulsion. The obtained primary emulsion was transferred to a high-pressure homogenizer for high-pressure homogenization, the homogenization pressure was 1000 bar, and the homogenization times was 20 times. The organic solvent was removed from the homogenized system by rotary evaporation. The rotary-evaporated medicinal solution was filtered and sterilized via a 0.22 μm microporous membrane to obtain an aqueous solution of elemene albumin nanoparticles. 3% trehalose was added for lyophilization, capped after lyophilization, and stored at 4°C. The lyophilized sample was taken and reconstituted in water for injection, and the measured particle size was 112.6 nm, PDI was 0.101.

**Example 13**

500 mg of β-elemene and 1200 mg of soybean oil were weighed, successively added to isopropanol (5 mL) and mixed by vortex to obtain an oil phase. 1500 mg of bovine serum albumin was taken and dissolved in water for injection to obtain an aqueous solution of bovine serum albumin with a mass volume percentage of 3% (g/mL), to obtain an aqueous phase. The oil phase was added dropwise to the aqueous phase, and sonicated by a probe for 8 min (250 W) to produce a primary emulsion. The obtained primary emulsion was transferred to a high-pressure homogenizer for high-pressure homogenization, the homogenization pressure was 1400 bar, and the homogenization times was 10 times. The organic solvent was removed from the homogenized system by rotary evaporation. The rotary-evaporated medicinal solution was filtered and sterilized via a 0.22 μm microporous membrane. The filtrate was diluted and configured to a drug concentration of 10 mg/mL, and 3% sucrose was added for lyophilization, capped after lyophilization, and stored at 4°C. The lyophilized samples were reconstituted in water for injection, and the measured particle size was 128.1 nm, PDI was 0.132.

**Example 14**

500 mg of β-elemene and 1200 mg of soybean oil were weighed, successively added to tertiary butanol (5 mL) and mixed by vortex to obtain an oil phase. 1500 mg of bovine serum albumin was taken and dissolved in water for injection to obtain an aqueous solution of bovine serum albumin with a mass volume percentage of 3% (g/mL), to obtain an aqueous phase. The oil phase was added dropwise to the aqueous phase, and sonicated by a probe for 8 min (250 W) to produce a primary emulsion. The obtained primary emulsion was transferred to a high-pressure homogenizer for high-pressure homogenization, the homogenization pressure was 1400 bar, and the homogenization times was 10 times. After homogenization, the organic solvent was removed by rotary evaporation. The rotary-evaporated medicinal solution was filtered and sterilized via a 0.22 μm microporous membrane. The filtrate was diluted and configured to a drug concentration of 5 mg/mL, and 3% sucrose was added for lyophilization, capped after lyophilization, and stored at 4°C. The lyophilized sample was taken and reconstituted in water for injection, and the measured particle size was 116.8 nm, PDI was 0.113.

**Example 15**

500 mg of β-elemene and 1200 mg of sesame oil were weighed, successively added to a dichloromethane/isopropanol mixed solvent (dichloromethane:isopropanol=4:1, 5 mL), and mixed by vortex to obtain an oil phase. 1500 mg of bovine serum albumin was dissolved in water for injection to obtain an aqueous solution of bovine serum albumin with a mass volume percentage of 3% (g/mL), to obtain an aqueous phase. The oil phase was added dropwise to the aqueous phase, and sonicated by a probe for 8 min (250 W) to produce a primary emulsion. The obtained primary emulsion was transferred to a high-pressure homogenizer for high-pressure homogenization, the homogenization pressure was 1400 bar, and the homogenization times was 10 times. After homogenization, the organic solvent was removed by rotary evaporation. The rotary-evaporated medicinal solution was filtered and sterilized via a 0.22 μm microporous membrane. The filtrate was diluted and configured to a drug concentration of 1 mg/mL, and 3% sucrose was added for lyophilization, capped after lyophilization, and stored at 4°C. The lyophilized sample was taken and reconstituted in water for injection, and the measured particle size was 121.8 nm, PDI was 0.111.

**Example 16**

500 mg of β-elemene and 1200 mg of soybean oil were weighed, successively added to a dichloromethane/-tertiary butanol mixed solvent (dichloromethane:tertiary butanol=4:1, 5 mL), and mixed by vortex to obtain an oil phase. 1500 mg of bovine serum albumin was dissolved in water for injection to obtain an aqueous solution of bovine serum albumin with a mass volume percentage of 3% (g/mL), to obtain an aqueous phase. The oil phase was added dropwise to the aqueous phase, and sonicated by a probe for 8 min (250 W) to produce a primary emulsion. The obtained primary emulsion was transferred to a high-pressure homogenizer for high-pressure homogenization, the homogenization pressure was 1400 bar, and the homogenization times was 10 times. After homogenization, the organic solvent was removed by rotary evaporation. The rotary-evaporated medicinal solution was filtered and sterilized via a 0.22 μm microporous membrane. The filtrate was diluted and configured to a drug concentration of 10 mg/mL, and 3% sucrose was added for lyophilization, capped after lyophilization, and stored at 4°C. The lyophilized sample was taken and reconstituted in water for injection, and the measured particle size was 121.1 nm, PDI was 0.109.

**Example 17**

500 mg of β-elemene and 1200 mg of olive oil were weighed, successively added to a dichloromethane/ethanol mixed solvent (dichloromethane:ethanol=4:1, 5 mL), and mixed by vortex to obtain an oil phase. 1500 mg of bovine serum albumin was dissolved in water for injection to obtain an aqueous solution of bovine serum albumin with 3% of mass volume percentage (g/mL), to obtain an aqueous phase. The oil phase was added dropwise to the aqueous phase, and sonicated by a probe for 8 min (250 W) to produce a primary emulsion. The obtained primary emulsion was transferred to a high-pressure homogenizer for high-pressure homogenization, the homogenization pressure was 1400 bar, and the homogenization times was 10 times. The organic solvent was removed from the homogenized system by rotary evaporation. The rotary-evaporated medicinal solution was filtered and sterilized via a 0.22 μm microporous membrane. The filtrate was diluted and configured to a drug concentration of 12 mg/mL, and 3% sucrose was added for lyophilization, capped after lyophilization, and stored at 4°C. The lyophilized sample was taken and reconstituted in water for injection, and the measured particle size was 109.7 nm, PDI was 0.105.

**Example 18**

500 mg of β-elemene and 1200 mg of soybean oil were weighed, successively added to a dichloromethane/tetrahydrofuran mixed solvent (dichloromethane:tetrahydrofuran=4:1, 5 mL), and mixed by vortex, to obtain an oil phase. 1500 mg of bovine serum albumin was dissolved in water for injection to obtain an aqueous solution of bovine serum albumin with 3% of mass volume percentage (g/mL), to obtain an aqueous phase. The oil phase was added dropwise to the aqueous phase, and sonicated by a probe for 8 min (250 W) to produce a primary emulsion. The obtained primary emulsion was transferred to a high-pressure homogenizer for high-pressure homogenization, the homogenization pressure was 1400 bar, and the homogenization times was 10 times. The organic solvent was removed from the homogenized system by rotary evaporation. The rotary-evaporated medicinal solution was filtered and sterilized via a 0.22 μm microporous membrane. The filtrate was diluted and configured to a drug concentration of 10 mg/mL, and 3% sucrose was added for lyophilization, capped after lyophilization, and stored at 4°C. The lyophilized sample was taken and reconstituted in water for injection, and the measured particle size was 112.7 nm, PDI was 0.112.

## Example 19

200 mg of elemene and 600 mg of soybean oil were weighed, dissolved in a mixed solvent of dichloromethane and ethanol (dichloromethane:ethanol=4:1, 2 mL), and mixed by vortex, to obtain an oil phase. 650 mg of human serum albumin was dissolved in 18 mL of 0.5% sodium chloride solution to obtain a solution of human serum albumin with a mass volume percentage (g/mL) of 3.6%. Under the action of water bath ultrasound, the oil phase was added dropwise to the aqueous phase, and sonicated by a probe for 8 min (250 W) to produce a primary emulsion. The obtained primary emulsion was transferred to a high-pressure homogenizer for high-pressure homogenization, the homogenization pressure was 1000 bar, and the homogenization times was 16 times. The organic solvent was removed from the homogenized system by rotary evaporation, and a 0.22 μm microporous membrane was used for filtration and sterilization to obtain an aqueous solution of elemene albumin nanoparticles. The measured particle size was 126.1 nm, PDI was 0.122.

## Example 20

500 mg of β-elemene and 1500 mg of soybean oil were weighed, dissolved in 5 mL of dichloromethane/ethanol (dichloromethane:ethanol=8:1) mixed solvent to obtain an oil phase; and 2500 mg of human serum albumin was weighed and dissolved in 45 mL of water to obtain an aqueous phase. Under the action of ultrasound, the two phases were mixed evenly, and the obtained solution was transferred to a high-pressure homogenizer for high-pressure homogenization, at 400-500 bar for 4-6 times, and at 1300-1400 bar for 8-10 times. After homogenization, then the organic solvent was removed by rotary evaporation, the microporous membrane was used for filtration and sterilization, the bubbles in the formulation were removed by standing still for a moment, and a solution of elemene albumin nanoparticles was obtained. The measured particle size of the nanoparticles was 99.4 nm, PDI was 0.096. The obtained solution of elemene albumin nanoparticles was adjusted to a concentration of 10 mg/mL, 3% sucrose was added, lyophilized, and reconstituted by adding ultrapure water, the particle size was measured to be 101.6 nm, and the PDI was 0.113.

## Example 21

The corresponding β-elemene, soybean oil, MCT (medium chain triglyceride) and surfactants (HS-15, namely macrogol 15-hydroxystearate; Tween 80; Poloxamer P188) were weighed according to the component amounts listed in Table 5 and mixed to obtain an oil phase. 20% human serum albumin (converted by mass as shown in Table 5) was taken, diluted by adding ultrapure water to a total volume of 50 mL and mixed evenly, to obtain an aqueous solution of human serum albumin, namely an aqueous phase. An appropriate amount of surfactant was added to the aqueous phase solution, and the aqueous phase solution was poured into the oil phase, and sheared with a mixer (IKA, T25) at 5,000 rpm for 1 min and at 10,000 rpm for 5 min to form a primary emulsion. The obtained primary emulsion was transferred to a high-pressure homogenizer (ATS, AH-1500) for high-pressure homogenization at a homogenization pressure of 250-300 bar for 2 min, and at 1450-1550 bar for 6 min. After homogenization, the total volume of the homogeneous solution was measured with a measuring cylinder, trehalose of 10% (g/mL) was added, shaken well to be dissolved, and filtered with a 0.45μm microporous membrane to obtain a solution of elemene albumin nanoparticles. The particle size and PDI were detected, and the hemolysis value was determined. The specific results are shown in Table 5.

**Table 5**

| Serial number | β-elemene /g | Soybean oil /g | MCT /g | HSA/g | Soybean oil/API (g/g) | HSA/API (g/g) | Surfactant | Hemolysis value | Particle size (nm) | PDI |
|---|---|---|---|---|---|---|---|---|---|---|
| 1 | 0.5 | 0.6 | 0.6g | 1.5 | 1.2 | 3 | 0.1g HS-15 | 31.6 | 182.7 | 0.156 |
| 2 | 0.5 | 0.6 | 0.6g | 1.5 | 1.2 | 3 | 0.1g Tween 80 | 22.2 | 178.3 | 0.135 |
| 3 | 0.5 | 0.6 | 0.6g | 1.5 | 1.2 | 3 | 0.1g Poloxamer P188 | 53.3 | 232.5 | 0.143 |
| 4 | 1 | 0.4 | 0.1g | 1.5 | 0.4 | 1.5 | / | 6.4 | 128.3 | 0.167 |
| 5 | 1 | 0.6 | 0.15g | 1.5 | 0.6 | 1.5 | / | 9.1 | 127.6 | 0.191 |
| 6 | 1 | 0.4 | 0.1g | 5 | 0.4 | 5 | / | 13.8 | 103.4 | 0.162 |
| 7 | 1 | 0.6 | 0.15g | 5 | 0.6 | 5 | / | 7 | 101.2 | 0.092 |

**Example 22**

β-elemene and soybean oil were weighed according to the component amounts listed in Table 6 and mixed evenly, to obtain an oil phase. Human serum albumin (converted by mass as shown in Table 6) was measured, diluted to a total volume of 50 mL by adding ultrapure water and mixed evenly, to obtain an aqueous solution of human serum albumin, namely an aqueous phase. The aqueous phase was poured into the oil phase, and sheared with a mixer (IKA, T25) at 5,000 rpm for 1 min and at 10,000 rpm for 5 min to form a primary emulsion. The obtained primary emulsion was transferred to a high-pressure homogenizer (ATS, AH-1500) for high-pressure homogenization at a homogenization pressure of 250-300 bar for 2 min, and at 1450-1550 bar for 6min. After homogenization, the total volume of the homogeneous solution was measured with a measuring cylinder, trehalose was added at a mass/volume ratio of 10% (g/mL), shaken well to be dissolved, and filtered with a 0.45μm microporous membrane to obtain a solution of elemene albumin nanoparticles. The particle size and PDI were detected, and the hemolysis value was determined. The specific results are shown in Table 6.

Table 6

| Serial number | β-elemene /g | Soybean oil /g | HSA /g | Soybean oil/API (g/g) | HSA/API (g/g) | Hemolysi s value | Particle size (nm) | PDI |
|---|---|---|---|---|---|---|---|---|
| 1 | 1 | 0.5 | 1.5 | 0.5 | 1.5 | 7.8 | 130.1 | 0.161 |
| 2 | 1 | 0.75 | 1.5 | 0.75 | 1.5 | 5.2 | 132 | 0.16 |
| 3 | 1 | 0.5 | 5 | 0.5 | 5 | 8.3 | 111 | 0.084 |
| 4 | 1 | 0.75 | 5 | 0.75 | 5 | 3.8 | 111.8 | 0.071 |
| 5 | 1 | 0.5 | 10 | 0.5 | 10 | 13.7 | 94.99 | 0.107 |
| 6 | 0.5 | 1.2 | 1.5 | 2.4 | 3 | 15.1 | 130.3 | 0.079 |
| 7 | 0.5 | 1.2 | 5 | 2.4 | 10 | 13.1 | 131.1 | 0.08 |
| 8 | 0.5 | 1.2 | 7.5 | 2.4 | 15 | 11.7 | 132.9 | 0.085 |
| 9 | 0.5 | 1.2 | 10 | 2.4 | 20 | 13.5 | 127.3 | 0.067 |
| 10 | 0.5 | 1.2 | 1.5 | 2.4 | 3 | 11 | 143.2 | 0.161 |
| 11 | 1 | 0.35 | 1.5 | 0.35 | 1.5 | 9 | 115.43 | 0.132 |

**Example 23**

3.33 g of β-elemene and 8g of soybean oil were weighed and mixed to obtain an oil phase. 50 mL (10g) of 20% human serum albumin (HSA) was measured, diluted evenly by adding an appropriate amount of ultrapure water, to obtain an aqueous solution of human serum albumin with 3% of mass volume percentage (g/mL), namely an aqueous phase. The aqueous phase solution was poured into the oil phase, and the mixer (FLUKO, FA30/30F) was used for shearing at 10,000 rpm for 10 min to form a primary emulsion. The obtained primary emulsion was transferred to a micro-jet homogenizer (Microfluidizer, M-110-EH) for homogenization, at a homogenization pressure of 20,000 psi for 30 min. The homogenized solution was filtered by 0.45 μm and 0.22 μm PVDF plate filters, and the metered volume was adjusted, and different lyoprotectants (g/mL) corresponding to Table 7 was added respectively. The homogeneous solution added with the lyoprotectant was lyophilized, and after lyophilization, the lyophilized powder was reconstituted with sterilized ultrapure water. The particle size, PDI and hemolysis value were detected. The specific results are shown in Table 7.

Table 7

| Serial number | Prescription | Protectant | Hemolysis value | Particle size | PDI |
|---|---|---|---|---|---|
| 1 | | 3% Sucrose | 13.4 | 175.97 | 0.188 |
| 2 | 3.33 g β-elemene 8g Soybean oil 10g HSA | 10% Sucrose | 13.4 | 138.76 | 0.119 |
| 3 | | 3% Trehalose | 11.3 | 170.91 | 0.192 |
| 4 | | 10% Trehalose | 14 | 138.39 | 0.158 |

**Example 24**

3.33 g of β-elemene and 8 g of soybean oil were weighed, dissolved in an organic solvent, and mixed to obtain an oil phase. The organic solvent was a mixed solvent of 26.67 mL dichloromethane and 6.67 mL anhydrous ethanol. 50 mL (10g) of 20% human serum albumin (HSA) was measured, diluted evenly by adding an appropriate amount of ultrapure water, to obtain an aqueous solution of human serum albumin with a mass volume percentage of 3% (g/mL), namely an aqueous phase. The aqueous phase solution was poured into the oil phase, and the mixer (FLUKO, FA30/30F) was used for shearing at 10,000 rpm for 10 min to form a primary emulsion. The obtained primary emulsion was transferred to a micro-jet homogenizer (Microfluidizer, M-110-EH) for high-pressure homogenization, at a homogenization pressure of 20,000 psi for 30 min. After homogenization, the organic solvent was removed by a thin film evaporator from the organic solvent-containing emulsion, and the parameters were: temperature 30°C, vacuum degree 4000 pa, liquid feeding speed 100 rpm, scraper speed 50 rpm. The obtained emulsion was filtered by 0.45 μm and 0.22 μm PVDF plate filters, and the metered volume was adjusted, and 10% (g/mL) of trehalose was added as a lyoprotectant. The homogeneous solution added with the lyoprotectant was lyophilized, and after lyophilization, the lyophilized powder was reconstituted with sterilized ultrapure water. The detected particle size was 76.28, the PDI was 0.254 and the hemolysis value was 16.3.

**Example 25**

β-elemene and soybean oil were weighed according to the component amounts listed in Table 8, and dissolved in an organic solvent, the organic solvent was a mixed solvent of 1 mL of ethanol and 4 mL of dichloromethane, and mixed to obtain an oil phase. An appropriate amount of 20% human serum albumin (HSA) (converted by mass as shown in Table 8) was measured, diluted to a total volume of 50 mL by adding ultrapure water, namely an aqueous phase. The aqueous phase solution was poured into the oil phase, and sheared with a mixer (IKA, T25) at 5,000 rpm for 1 min and 10,000 rpm for 5 min to form a primary emulsion. The obtained primary emulsion was transferred to a high-pressure homogenizer (ATS, AH-1500) for high-pressure homogenization at a homogenization pressure of 250-300 bar for 2 min, and at 1450-1550 bar for 6min. The organic solvent was removed with a rotary evaporator at 35°C, 150 rpm and a vacuum of 30 mbar. After completion, the total volume of the homogeneous solution was measured with a measuring cylinder, trehalose was added at a mass/volume ratio of 10% (g/mL), shaken well to be dissolved, and filtered with a 0.45μm microporous membrane to obtain a solution of elemene albumin nanoparticles.

**Table 8**

| Serial number | β-elemene /g | Soybean oil /g | HSA /g | Soybean oil/API (g/g) | HSA/API (g/g) |
|---|---|---|---|---|---|
| 1* | 0.5 | 0.1 | 1.5 | 0.2 | 3 |
| 2 | 0.5 | 1.2 | 1.5 | 2.4 | 3 |
| 3* | 0.5 | 2.5 | 1.5 | 5 | 3 |
| 4* | 0.5 | 5 | 1.5 | 10 | 3 |
| 5 | 0.5 | 1.2 | 2.5 | 2.4 | 5 |
| 6 | 0.5 | 1.2 | 5 | 2.4 | 10 |
| 7 | 0.5 | 1.2 | 10 | 2.4 | 20 |
| *Serial numbers 1, 3 and 4 do not form part of the invention. | | | | | |

The prepared samples were placed in an oven at 30°C, and sampled at 0h, 24h, and 48h to determine particle size, PDI and β-elemene content. The measurement results are shown in Table 9.

**Table 9**

| Serial number | 0h | | 24h | | | 48h | | |
|---|---|---|---|---|---|---|---|---|
| | Particle size | PDI | Particle size | PDI | Content change (%) | Particle size | PDI | Content change (%) |
| 1 | 326.9 | 0.07 | 348.5 | 0.177 | -10.14 | 317.8 | 0.094 | -18.84 |
| 2 | 79.11 | 0.14 | 80.22 | 0.229 | -3.19 | 77.04 | 0.164 | -5.32 |
| 3 | 99.59 | 0.174 | 101.6 | 0.203 | -2.56 | 101.7 | 0.183 | -3.85 |

(continued)

| Serial number | 0h | | 24h | | | 48h | | |
|---|---|---|---|---|---|---|---|---|
| | Particle size | PDI | Particle size | PDI | Content change (%) | Particle size | PDI | Content change (%) |
| 4 | 121.8 | 0.194 | 125.4 | 0.211 | 0 | 119 | 0.216 | -1.16 |
| 5 | 88.17 | 0.187 | 97.63 | 0.237 | -2.35 | 99.19 | 0.219 | -4.71 |
| 6 | 82.47 | 0.259 | 99.54 | 0.362 | -3.70 | 102.6 | 0.271 | -6.17 |
| 7 | 77.82 | 0.283 | 102.9 | 0.261 | -2.0 | 104 | 0.22 | -8.69 |

**Example 26**

β-elemene and soybean oil were weighed according to the component amounts listed in Table 10, to obtain an oil phase. An appropriate amount of 20% human serum albumin (HSA) (converted by mass as shown in Table 10) was measured, diluted to a total volume of 50 mL by adding ultrapure water, namely an aqueous phase. The aqueous phase solution was poured into the oil phase, and sheared with a mixer (IKA, T25) at 5,000 rpm for 1 min and at 10,000 rpm for 5 min to form a primary emulsion. The obtained primary emulsion was transferred to a high-pressure homogenizer (ATS, AH-1500) for high-pressure homogenization at a homogenization pressure of 250-300 bar for 2 min, and at 1350-1450 bar for 6 min. After completion, the total volume of the homogeneous solution was measured with a measuring cylinder, trehalose was added at the mass/volume ratio of (g/mL) shown in Table 10, shaken well to be dissolved, and filtered with a 0.45μm microporous membrane to obtain a solution of elemene albumin nanoparticles.

**Table 10**

| Serial number | β-elemene/g | Soybean oil /g | HSA /g | Soybean oil/API (g/g) | HSA/API (g/g) | Protectant (g/mL) |
|---|---|---|---|---|---|---|
| 1 | 1 | 1 | 1.5 | 1 | 1.5 | 10% Trehalose |
| 2 | 1 | 1.5 | 1.5 | 1.5 | 1.5 | 10% Trehalose |
| 3 | 1 | 2 | 1.5 | 2 | 1.5 | 10% Trehalose |
| 4 | 1 | 2.5 | 1.5 | 2.5 | 1.5 | 10% Trehalose |
| 5 | 1 | 3 | 1.5 | 3 | 1.5 | 10% Trehalose |
| 6 | 1 | 0.5 | 0.5 | 0.5 | 0.5 | 10% Trehalose |
| 7 | 1 | 0.5 | 1 | 0.5 | 1 | 10% Trehalose |
| 8 | 1 | 0.5 | 1.5 | 0.5 | 1.5 | 10% Trehalose |
| 9 | 1 | 0.5 | 2 | 0.5 | 2 | 10% Trehalose |
| 10 | 1 | 0.5 | 2.5 | 0.5 | 2.5 | 10% Trehalose |
| 11 | 1 | 0.5 | 1.5 | 0.5 | 1.5 | 20% Trehalose |
| 12 | 1 | 0.5 | 1.5 | 0.5 | 1.5 | 15% Trehalose |
| 13 | 1 | 0.5 | 1.5 | 0.5 | 1.5 | 5% Trehalose |

The prepared samples were placed in an oven at 30°C, and sampled at 0h, 24h, and 48h to determine particle size, PDI and β-elemene content. The measurement results are shown in Table 11.

**Table 11**

| Serial number | 0h | | 24h | | | 48h | | |
|---|---|---|---|---|---|---|---|---|
| | Particle size | PDI | Particle size | PDI | Content change (%) | Particle size | PDI | Content change (%) |
| 1 | 140.3 | 0.127 | 143.7 | 0.147 | -3.60 | 135.5 | 0.165 | -5.76 |
| 2 | 146.7 | 0.204 | 154.2 | 0.226 | -3.64 | 149.5 | 0.208 | -4.24 |

(continued)

| Serial number | 0h | | 24h | | | 48h | | |
|---|---|---|---|---|---|---|---|---|
| | Particle size | PDI | Particle size | PDI | Content change (%) | Particle size | PDI | Content change (%) |
| 3 | 141.7 | 0.213 | 144.6 | 0.251 | -1.83 | 141.4 | 0.265 | -2.44 |
| 4 | 131.8 | 0.192 | 135.9 | 0.229 | -3.05 | 132.3 | 0.222 | -1.83 |
| 5 | 105.8 | 0.31 | 117.4 | 0.36 | -1.24 | 106.8 | 0.285 | -1.24 |
| 6 | 152.3 | 0.188 | 154.5 | 0.122 | -3.59 | 151.3 | 0.13 | -3.59 |
| 7 | 143.2 | 0.108 | 144.5 | 0.139 | -3.85 | 142.2 | 0.129 | -5.13 |
| 8 | 123.2 | 0.059 | 125.6 | 0.121 | -4.19 | 121.6 | 0.089 | -5.39 |
| 9 | 117.3 | 0.15 | 117.3 | 0.132 | -4.79 | 116.8 | 0.126 | -7.19 |
| 10 | 154.1 | 0.145 | 159.2 | 0.185 | -4.17 | 163.5 | 0.167 | -6.55 |
| 11 | 133.7 | 0.095 | 134.1 | 0.122 | -4.61 | 131.5 | 0.118 | -5.93 |
| 12 | 139.9 | 0.097 | 134 | 0.115 | -5.63 | 128.4 | 0.194 | -6.88 |
| 13 | 130.6 | 0.132 | 122.2 | 0.115 | -4.14 | 128.7 | 0.159 | -5.33 |

## Example 27

2g of β-elemene and 1.5g of soybean oil were weighed and mixed to obtain an oil phase. 45 mL (9 g) of 20% human serum albumin was measured, namely an aqueous phase. The aqueous phase solution was poured into the oil phase, and sheared with a mixer (IKA, T25) at 5,000 rpm for 1 min and 10,000 rpm for 5 min to form a primary emulsion. The obtained primary emulsion was transferred to a high-pressure homogenizer (ATS, AH-1500) for high-pressure homogenization at a homogenization pressure of 250-300 bar for 2 min, and at 1450-1550 bar for 6 min, and the homogeneous solution was collected. The pipeline of the homogenizer was flushed with an aqueous solution of trehalose (25 mL of water + 10 g of trehalose) to replace the homogeneous solution remaining in the homogenizer, and then the pipeline of the homogenizer was flushed with 30 mL of ultrapure water to replace the residual aqueous solution of trehalose. The trehalose aqueous solution was combined with the homogeneous solution, mixed, and filtered through a 0.22 μm sterile filter membrane. The particle size was 106.4 nm detected by a particle size analyzer, PDI was 0.08.

## Example 28

**Table 13**

| Serial number | Prescription (g) | | | Soybean/API (g/g) | HSA/API (g/g) | Protectant (g/mL) |
|---|---|---|---|---|---|---|
| | API | Soybean oil | HSA | | | |
| 1 | 2 | 2 | 10 | 1 | 5 | 10% Trehalose |
| 2 | 2 | 3 | 10 | 1.5 | 5 | 10% Trehalose |
| 3 | 2 | 4 | 10 | 2 | 5 | 10% Trehalose |
| 4 | 2 | 5 | 10 | 2.5 | 5 | 10% Trehalose |
| 5 | 3 | 4.5 | 9 | 1.5 | 3 | 10% Trehalose |

β-elemene and soybean oil were weighed as shown in Table 13 and mixed to obtain an oil phase. 20% human serum albumin (50 mL, 10 g) was measured, namely an aqueous phase. The aqueous phase solution was poured into the oil phase, and sheared with a mixer (IKA, T25) at 5,000 rpm for 1 min and at 10,000 rpm for 5 min to form a primary emulsion. The obtained solution was transferred to a high-pressure homogenizer (ATS, AH-1500) for high-pressure homogenization at a homogenization pressure of 250-300 bar for 2 min, and at 1550-1550 bar for 6 min. 10 g of trehalose was weighed and dissolved to 50 mL by adding pure water (in which sample No. 5 was added with 15 g of trehalose and dissolved to 105 mL by adding pure water). The pipeline of the homogenizer was flushed with the aqueous solution of trehalose to replace the homogeneous solution remaining in the homogenizer, and the residual homogeneous solution was combined with the homogeneous solution, mixed, and filtered through a 0.45 $\mu$m sterile filter membrane. Each sample was tested for hemolysis value. And the sample was divided into EP tubes, stored in a thermotank at 30°C, and the samples were taken at 0h, 24h, and 48h to determine particle size, PDI and $\beta$-elemene content. The results are shown in Table 14.

**Table 14**

| Serial number | 0h | | 24h | | | 48h | | | |
|---|---|---|---|---|---|---|---|---|---|
| | Particle size | PDI | Particle size | PDI | Content change % | Particle size | PDI | Content change % | Hemolysis value |
| 1 | 102.9 | 0.124 | 99.7 | 0.090 | -4.22% | 99.8 | 0.085 | -5.42% | 6.0 |
| 2 | 113.7 | 0.112 | 108.2 | 0.095 | -1.81% | 107.9 | 0.082 | -4.82% | 9.3 |
| 3 | 120.9 | 0.034 | 128.9 | 0.052 | -2.33% | 128.3 | 0.106 | -4.07% | 17.4 |
| 4 | 126.4 | 0.070 | 126.4 | 0.168 | -2.33% | 132.4 | 0.138 | -2.91% | 16.5 |
| 5 | 111.8 | 0.089 | 111.0 | 0.108 | -5.52% | 109.1 | 0.066 | -6.08% | 10.2 |

**Example 29**

2g of $\beta$-elemene and 1.5g of soybean oil were weighed and mixed to obtain an oil phase. 50 mL (10 g) of 20% human serum albumin was measured, namely an aqueous phase. The aqueous phase solution was poured into the oil phase, and sheared with a mixer (IKA, T25) at 5,000 rpm for 1 min and at 10,000 rpm for 5 min to form a primary emulsion. And 5 mL of water for injection was used for cleaning the shearing head, and combined with the primary emulsion. The obtained solution was transferred to a high-pressure homogenizer (ATS, AH-1500) for high-pressure homogenization at a homogenization pressure of 250-300 bar for 2 min, and at 1500-1550 bar for 6 min, and the homogeneous solution was collected. The pipeline of the homogenizer was flushed with 22.5 mL of an aqueous solution of trehalose to replace the homogeneous solution remaining in the homogenizer, and then the pipeline of the homogenizer was flushed with 22.5 mL of ultrapure water to replace the residual aqueous solution of trehalose. The trehalose solution was combined with the homogeneous solution, mixed, and filtered via a 0.22 $\mu$m sterile filter membrane. The sample was divided into EP tubes, stored in refrigerator at 2-8°C, and the particle size and PDI were detected by sampling on the 0th day and second month, respectively. The results are shown in Table 12.

**Table 12**

| Time | Particle size | PDI |
|---|---|---|
| Day 0 | 87.93 | 0.107 |
| 2nd month | 89.39 | 0.119 |

**Example 30 Drug Efficacy Study**

**Preparation of sample**

4.5 g of $\beta$-elemene and 10.8 g of soybean oil were weighed and mixed to obtain an oil phase. 4 mL of ethanol and 16 mL of dichloromethane were measured, and mixed evenly to obtain organic phase A. The oil phase was added to the organic phase and the metered volume was adjusted to 36 mL to obtain organic phase B.

13.5 g of human serum albumin was weighed and added to 405 mL of purified water, stirred to be dissolved. The organic phase B was added to the aqueous phase, and stirred with a high-speed shearing machine at gear A for 2 min, and then at gear B for 1 min to obtain primary emulsion A. The primary emulsion A was divided into 3 parts, and treated by a cell disrupter at 250w for 8 min to obtain primary emulsion B. The primary emulsion B was added into a high-pressure homogenizer, firstly at 200 bar for 3 cycles, and then at 1300 bar for 5 cycles to obtain albumin nanoparticles. The above nanoparticles were concentrated under reduced pressure at 37°C for 1 h, added with 12.30 g of 3% sucrose, filtered at 0.22 $\mu$m, and lyophilized. The particle size was 81.03 nm, PDI was 0.174.

**Experimental Methods:**

Establishment of a human-derived brain tumor xenograft mouse model: 50 nude mice were anesthetized with 2.5% isoflurane, a small hole (<0.5 mm) was drilled in the skull of the nude mice, and cells expressing the luminescent reporter gene (5 × 10$^4$ U87MGR1 Human glioma cells) were injected into the left forehead (2.5 mm lateral and 0.5 mm anterior bregma, 2.5 mm deep).

In vivo bioluminescence imaging assay method: nude mice received a single subcutaneous injection of D-luciferin solution (120 mg/kg). 10 min after injection of fluorescein, light images of animals were taken with dark multicolor illumination during an exposure time of 1-5 min (for bioluminescence imaging of Gluc, a single intravenous injection of coelenterazine (20 mg/kg, at total volume of 150 μL) was given). Intratumoral luciferase activity was obtained by recording photon counts with a CCD camera over 5 min without lighting. Image intensity was quantitatively analyzed using Perkin Elmer's Living image software 4.3.1.

One week after inoculation, in vivo bioluminescence imaging was performed to evaluate the tumor volume in nude mice. 32 nude mice with similar tumor volume were selected and randomly divided into 8 mice (half male and half female)/group, and the dose were administered by tail vein injection for 3 weeks, 3 times/week:

1) model group (control) (0.1% DMSO prepared by PBS), n=8;
2) 50 mg/kg temozolomide (TMZ), n=8;
3) 50 mg/kg Ailineng (ELE mic), n=8;
4) 50 mg/kg (Alb), n=8;

Tumor volume was measured weekly.

During the experiment, the animals were weighed weekly to monitor their healthy conditions.

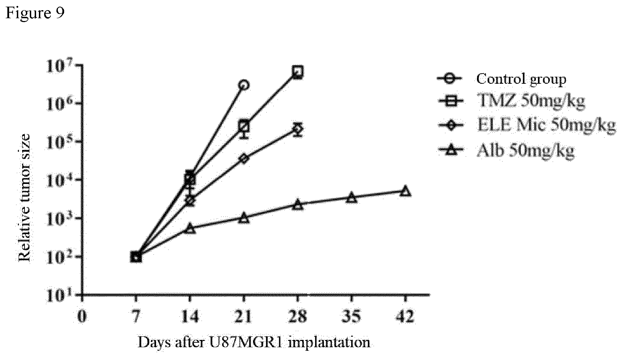

Dosing was discontinued after two weeks of administration, followed by an observation for three weeks. The survival and tumor volume of mice in each group were recorded using Graphpad software to generate Kaplan-Meier plots. The tumor growth curve of the mouse orthotopic tumor model is shown in Figure 8 (the tumor-bearing nude mice were treated with Alb formulation, 3 times/week × 2 weeks (n=8, half male and half female); compared with temozolomide (TMZ), p< 0.01), the survival curve of the mouse orthotopic tumor model is shown in Figure 9 (the tumor-bearing nude mice were treated with Alb, 3 times/week × 2 weeks (n=8, half male and half female); compared with temozolomide (TMZ), p <0.01).

**Example 31 Safety Study**

**Preparation of sample**

Elemene injection (trade name: Ailineng)): CSPC Yuanda (Dalian) Pharmaceutical Co., Ltd., batch number: 17050302)

β-elemene micelle formulation:

17.08 g of RH40 was weighed and heated at 60°C to be dissolved. 2 mL of β-elemene was added, heated and stirred at 60°C for 5 min. 14.40 g of propylene glycol and 38.72 g of purified water were weighed and mixed, then heated at 60°C for 10min. The aqueous solution of propylene glycol was added to the RH40 solution containing β-elemene, and stirred in a water bath at 60°C for 30 min to obtain β-elemene micelles. The above micelles were filtered at 0.22 μm, and divided into 5 mL/per formulation.

β-elemene albumin formulation:

2.5001 g of β-elemene and 6.001 g of soybean oil were weighed and mixed for later use. 2 mL of ethanol and 8 mL of dichloromethane were measured, and mixed evenly for later use. The volume of the oil phase was adjusted to 12 mL by the organic phase for later use.

4.5015 g of human serum albumin was weighed and added to 135 mL of purified water, stirred to be dissolved. The organic phase was added to the aqueous phase, and stirred with a high-speed shearing machine at gear A for 2 min, and then at gear B for 0.5 min to obtain primary emulsion A. The primary emulsion A was divided into 3 parts, and treated by a cell disrupter at 250W for 8 min to obtain primary emulsion B. The primary emulsion B was added into a high-pressure homogenizer, firstly at 200 bar for 3 cycles, and then at 1300-1400 bar for 5 cycles to obtain albumin nanoparticles. The above nanoparticles were concentrated under a reduced pressure at 37°C for 1 h, added with 1.20 g of sucrose, filtered at 0.22 μm, and lyophilized.

**Solution Preparation:**

The three formulations were prepared into 6 mg/mL solution using normal saline.

Ailineng: 7.5 mL of 20 mg/mL commercially available Ailineng was taken and diluted into 25 mL of 6 mg/mL sample solution with 17.5 mL of normal saline.

β-elemene micelle formulation: 5.91 mL of 25.41 mg/mL β-elemene micelle formulation was taken and diluted into 25 mL of 6 mg/mL sample solution with 19.09 mL of normal saline.

β-elemene albumin formulation: 12 β-elemene micelle formulations were taken, 2.01 mL of normal saline was added to each one, then mixed to obtain 24 mL of 6 mg/mL sample solution.

**Test Methods:**

Male KM mice, 20-25 g/mouse, were randomly divided into 13 groups according to body weight, with 7 mice in each group. The mice were grouped according to the prescribed dose by weight. The dosage of each sample was set as 100 mg/kg, 130 mg/kg, 170 mg/kg and 220 mg/kg, and another blank group was set. The mice were injected through tail vein, and the site of administration, reaction after administration and death conditions were observed after administration. The results of the study are shown in Table 15.

**Table 15 Safety studies of different samples**

| Dosage / Sample | 100 mg/kg | 130 mg/kg | 170 mg/kg | 220 mg/kg |
|---|---|---|---|---|
| Ailineng | reacted intensely; respiratory distress; motionless | reacted intensely; respiratory distress; lethargic, motionless; started to recover in about 5 min | 3 died; reacted intensely; respiratory distress; lethargic; motionless, occasionally ataxia | 6 died; respiratory distress, extremely low physical activity. |
| β-elemene micelle formulation | much less stresses than Ailineng group; a few with respiratory distress | much less stresses than Ailineng group; respiratory distress, activity decreasing, and started to recover in about 2 min | reacted intensely; respiratory distress; lethargic, motionless | 3 died; respiratory distress; motionless |
| β-elemene albumin formulation | no obvious discomfort | no obvious discomfort, occasional mild respiratory distress | no obvious discomfort, occasional mild respiratory distress | no obvious discomfort, occasional respiratory distress |

**Claims**

1. A pharmaceutical composition comprising elemene, an oil for injection and a protein carrier, wherein the oil for injection is soybean oil and the protein carrier is human serum albumin, and wherein the weight ratio of the soybean oil to the elemene is about 0.35-3 and the weight ratio of the human serum albumin to the elemene is about 0.5-20.

2. The pharmaceutical composition according to claim 1, wherein the pharmaceutical composition is in the form of particles, and the particle size of the particles is less than 180 nm, preferably 50-180 nm, wherein the particle size is determined based on a 25 μL sample of the composition diluted with 1 mL of ultrapure water and using a MALVERN ZETASIZER NANO ZS90 particle size analyzer, a detection angle of 90° and at a temperature of 25°C.

3. The pharmaceutical composition according to claim 1, wherein the elemene is selected from one or more of $\alpha$-elemene, $\beta$-elemene, $\gamma$-elemene and $\delta$-elemene; preferably, the elemene is $\beta$-elemene.

4. The pharmaceutical composition according to any one of claims 1-3, further comprising a lyoprotectant; preferably, the lyoprotectant is selected from one or more of glucose, sucrose, maltose, lactose, mannose, trehalose, glycine and dextran.

5. The pharmaceutical composition according to any one of claims 1-4, further comprising one or more of isoosmotic adjusting agent, antioxidant, preservative, and pH regulator;

   preferably wherein the isoosmotic adjusting agent is one or more of glycerin, sorbitol, mannitol or glucose;
   or wherein the pH regulator is one or more of sodium hydroxide, sodium citrate, citric acid, phosphoric acid, acetic acid or hydrochloric acid;
   or wherein the preservative is selected from one or more of hydroxybenzene alkyl esters, benzoic acid, sodium benzoate, sorbic acid, chlorhexidine acetate, benzalkonium bromide;
   or wherein the antioxidant is selected from one or more of sodium sulfite, sodium bisulfite, sodium metabisulfite, sodium thiosulfate, ascorbic acid, butylated hydroxyanisole, 2,6-di-tert-butylated hydroxytoluene, and vitamin E.

6. The pharmaceutical composition according to any one of claims 1-5, wherein no organic solvent other than the oil for injection is included during preparation process thereof.

7. The pharmaceutical composition according to claim 6, wherein in the pharmaceutical composition, the weight ratio of the soybean oil to the elemene is about 0.5-3; and the weight ratio of the human serum albumin to the elemene is about 0.5-2.5;

   or wherein in the pharmaceutical composition, the weight ratio of the soybean oil to the elemene is about 0.35-1.5, preferably 0.35-0.75; and the weight ratio of the human serum albumin to the elemene is about 1.5-10;
   or wherein in the pharmaceutical composition, the weight ratio of the soybean oil to the elemene is about 0.5; and the weight ratio of the human serum albumin to the elemene is about 1.5;
   or wherein in the pharmaceutical composition, the weight ratio of the soybean oil to the elemene is about 0.75; and the weight ratio of the human serum albumin to the elemene is about 5;
   or wherein in the pharmaceutical composition, the weight ratio of the soybean oil to the elemene is about 1.5; and the weight ratio of the human serum albumin to the elemene is about 3-5.

8. The pharmaceutical composition according to any one of claims 1-5, wherein organic solvent is included during preparation process thereof;
   preferably wherein the organic solvent is selected from one or more of chloroform, dichloromethane, tertiary butanol, isopropanol, ethyl acetate, ethanol, tetrahydrofuran, dioxane, acetonitrile, acetone, dimethyl sulfoxide, dimethylformamide, and methylpyrrolidone.

9. The pharmaceutical composition according to claim 8, wherein the pharmaceutical composition is in the form of particles, and the particle size of the particles is about 70-150 nm, wherein the particle size is determined based on a 25 μL sample of the composition diluted with 1 mL of ultrapure water and using a MALVERN ZETASIZER NANO ZS90 particle size analyzer, a detection angle of 90° and at a temperature of 25°C;
   preferably, in the pharmaceutical composition, the weight ratio of the soybean oil to the elemene is about 2.4; and the weight ratio of the human serum albumin to the elemene is about 3.

**10.** A method for preparing the pharmaceutical composition of any one of claims 1-9, comprising the steps of:

(1) mixing elemene and an oil for injection evenly, optionally adding an organic solvent, to obtain a solution of the elemene and the oil for injection; dissolving a protein carrier in water to obtain a protein carrier solution;
(2) mixing the two solutions obtained in step (1) to form an emulsion;
(3) homogenizing the emulsion in step (2) under a high pressure, and optionally evaporating the emulsion under a reduced pressure to remove the organic solvent, to obtain a nanoparticle solution.

**11.** The method according to claim 10, wherein the organic solvent is used in step (1), preferably dissolving the elemene in the organic solvent and then mixing with the oil for injection;

or wherein the method further comprises a step of lyophilizing the obtained solution of protein carrier nanoparticles;
or wherein in step (2), a shearing or ultrasonic method is used for mixing; and in step (3), a high-pressure homogenization or micro-jet homogenization method is used for homogenization.

**12.** The pharmaceutical composition of any one of claims 1-9 for use in the prevention or treatment of cancer; preferably, the cancer is adrenocortical carcinoma, cryptogenic myeloid metaplasia, AIDS-related cancer, anal cancer, appendiceal cancer, astrocytoma, basal cell carcinoma, cholangiocarcinoma, bladder cancer, bone cancer, glioma, ependymoma, oligodendroglioma, meningioma, craniopharyngioma, hemangioblastoma, medulloblastoma, neuroectodermal tumor, visual pathway and hypothalamic glioma and glioblastoma, breast cancer, bronchial adenoma, carcinoid tumor, central nervous system lymphoma, cervical cancer, colon cancer, colorectal cancer, chronic myeloproliferative disorder, endometrial cancer, ependymoma, esophagus cancer, Ewing's tumor family, eye cancer, gallbladder cancer, gastrointestinal carcinoid tumor, gastrointestinal stromal tumor, germ cell carcinoma, gestational trophoblastic tumor, head and neck cancer, liver cancer, laryngeal cancer, leukemia, lip and oral cancer, lung cancer, lymphoma, medulloblastoma, melanoma, mesothelioma, metastatic neck squamous cell carcinoma, multiple endocrine neoplasia syndrome, myelodysplastic syndrome, myelodysplastic/myeloproliferative disease, carcinoma of nasal cavity and sinuses, nasopharyngeal cancer, neuroblastoma, neuroendocrine cancer, oropharyngeal cancer, brain tumor, bone metastasis cancer, gastric cancer, intestinal cancer, esophageal cancer, ovarian cancer, pancreatic cancer, breast cancer, skin cancer, parathyroid carcinoma, penile carcinoma, peritoneal cancer, pharyngeal carcinoma, pheochromocytoma, pineoblastoma and supratentorial primitive neuroectodermal tumor, pituitary tumor, pleuropulmonary blastoma, lymphoma, primary central nervous system lymphoma, pulmonary lymphangiomyomatosis, rectal cancer, kidney cancer, renal pelvis and ureter cancer, rhabdomyosarcoma, salivary gland cancer, skin cancer, small intestine cancer, squamous epithelial cell carcinoma, testicular cancer, throat cancer, thymoma and thymic carcinoma, thyroid cancer, urethral cancer, or vaginal cancer.

**13.** A pharmaceutical formulation, comprising the pharmaceutical composition of any one of claims 1-9.

**14.** The pharmaceutical formulation according to claim 13, wherein the pharmaceutical formulation is a solid formulation, a liquid formulation or a gas formulation;
preferably, the liquid formulation is a stable aqueous suspension reconstituted from a sterile lyophilized powder.

**15.** The pharmaceutical formulation according to claim 13, further comprising other drugs, including anticancer drugs; preferably, the anticancer drug comprises temozolomide, paclitaxel, docetaxel, taxane, gemcitabine, an anti-VEGF drug, or a PD-1 antibody drug.

**16.** A sealed container comprising the pharmaceutical composition of any one of claims 1-9 or the pharmaceutical formulation of any one of claims 13-15.

**Patentansprüche**

**1.** Pharmazeutische Zusammensetzung, die Elemene, ein Injektionsöl und einen Proteinträger umfasst, wobei das Injektionsöl Sojabohnenöl ist und der Proteinträger Humanserumalbumin ist, und wobei das Gewichtverhältnis des Sojabohnenöls zu Elemene etwa 0,35 bis 3 beträgt und das Gewichtverhältnis von Humanserumalbumin zu Elemene etwa 0,5 bis 20 beträgt.

**2.** Pharmazeutische Zusammensetzung nach Anspruch 1, wobei die pharmazeutische Zusammensetzung in der Form von Partikeln ist und die Partikelgröße der Partikel weniger als 180 nm beträgt, vorzugsweise 50-180 nm, wobei die Partikelgröße basierend auf einer 25-μL-Probe der Zusammensetzung, verdünnt mit 1 mL ultrapurem Wasser und unter Verwendung eines Partikelgrößenanalysators MALVERN ZETASIZER NANO ZS90 bei einem Detektionswinkel von 90° und bei einer Temperatur von 25 °C bestimmt wird.

3. Pharmazeutische Zusammensetzung nach Anspruch 1, wobei das Elemene ausgewählt ist aus einem oder mehreren von α-Elemene, β-Elemene, γ-Elemene und δ-Elemene ausgewählt ist; vorzugsweise ist das Elemene β-Elemene.

4. Pharmazeutische Zusammensetzung nach einem der Ansprüche 1-3, weiter umfassend ein Lyoprotektor; vorzugsweise ist der Lyoprotektor aus einem oder mehreren von Glucose, Sucrose, Maltose, Lactose, Mannose, Trehalose, Glycin und Dextran ausgewählt.

5. Pharmazeutische Zusammensetzung nach einem der Ansprüche 1-4, weiter umfassend eines oder mehrere von einem isoosmotischen Anpassungsmittel, Antioxidans, Konservierungsmittel und pH-Regulator;

   vorzugsweise wobei das isoosmotische Anpassungsmittel eines oder mehrere von Glycerin, Sorbitol, Mannitol oder Glucose ist;
   oder wobei der pH-Regulator eines oder mehrere von Natriumhydroxid, Natriumcitrat, Citronensäure, Phosphorsäure, Essigsäure oder Salzsäure ist;
   oder wobei das Konservierungsmittel aus einem oder mehreren von Hydroxybenzolalkylestern, Benzoesäure, Natriumbenzoat, Sorbinsäure, Chlorhexidinacetat, Benzalkoniumbromid ausgewählt ist;
   oder wobei das Antioxidans aus einem oder mehreren von Natriumsulfit, Natriumbisulfit, Natriummetabisulfit, Natriumthiosulfat, Ascorbinsäure, Butylhydroxyanisol, 2,6-di-tert-butylhydroxytoluol und Vitamin E ausgewählt ist.

6. Pharmazeutische Zusammensetzung nach einem der Ansprüche 1-5, während des Herstellungsprozesses kein anderes organisches Lösungsmittel als das Injektionsöl eingesetzt wird.

7. Pharmazeutische Zusammensetzung nach Anspruch 6, wobei in der pharmazeutischen Zusammensetzung das Gewichtsverhältnis von Sojabohnenöl zu Elemene etwa 0,5 bis 3 beträgt und das Gewichtsverhältnis von Humanserumalbumin zu Elemene etwa 0,5 bis 2,5 beträgt;

   oder wobei in der pharmazeutischen Zusammensetzung das Gewichtsverhältnis von Sojabohnenöl zu Elemene etwa 0,35 bis 1,5 beträgt, vorzugsweise 0,35 bis 0,75, und das Gewichtsverhältnis von Humanserumalbumin zu Elemene etwa 1,5 bis 10 beträgt;
   oder wobei in der pharmazeutischen Zusammensetzung das Gewichtsverhältnis von Sojabohnenöl zu Elemene etwa 0,5 beträgt und das Gewichtsverhältnis von Humanserumalbumin zu Elemene etwa 1,5 beträgt;
   oder wobei in der pharmazeutischen Zusammensetzung das Gewichtsverhältnis von Sojabohnenöl zu Elemene etwa 0,75 beträgt und das Gewichtsverhältnis von Humanserumalbumin zu Elemene etwa 5 beträgt;
   oder wobei in der pharmazeutischen Zusammensetzung das Gewichtsverhältnis von Sojabohnenöl zu Elemene etwa 1,5 beträgt und das Gewichtsverhältnis von Humanserumalbumin zu Elemene etwa 3 bis 5 beträgt;

8. Pharmazeutische Zusammensetzung nach einem der Ansprüche 1-5, wobei während des Herstellungsprozesses ein organisches Lösungsmittel eingesetzt wird;
   vorzugsweise wobei das organische Lösungsmittel aus einem oder mehreren von Chloroform, Dichlormethan, tert-Butanol, Isopropanol, Ethylacetat, Ethanol, Tetrahydrofuran, Dioxan, Acetonitril, Aceton, Dimethylsulfoxid, Dimethylformamid und Methylpyrrolidon ausgewählt ist.

9. Pharmazeutische Zusammensetzung nach Anspruch 8, wobei die pharmazeutische Zusammensetzung in der Form von Partikeln ist und die Partikelgröße der Partikel etwa 70 bis 150 nm beträgt, wobei die Partikelgröße basierend auf einer 25-μL-Probe der Zusammensetzung, verdünnt mit 1 mL ultrapurem Wasser und unter Verwendung eines Partikelgrößenanalysators MALVERN ZETASIZER NANO ZS90 bei einem Detektionswinkel von 90° und bei einer Temperatur von 25 °C bestimmt wird;
   vorzugsweise beträgt in der pharmazeutischen Zusammensetzung das Gewichtsverhältnis von Sojabohnenöl zu Elemene etwa 2,4 und das Gewichtsverhältnis von Humanserumalbumin zu Elemene etwa 3;

10. Herstellungsverfahren der pharmazeutischen Zusammensetzung nach einem der Ansprüche 1-9, umfassend die Schritte:

(1) gleichmäßiges Vermischen von Elemene mit einem Injektionsöl, wobei gegebenenfalls ein organisches Lösungsmittel zugesetzt wird, um eine Lösung aus Elemene und dem Injektionsöl zu erhalten; Auflösen eines Proteinträgers in Wasser, um eine Proteinträgerlösung zu erhalten;
(2) Vermischen der in Schritt (1) erhaltenen beiden Lösungen, um eine Emulsion zu erhalten;
(3) Homogenisieren der Emulsion in Schritt (2) unter einem hohen Druck und optional Verdampfen der Emulsion unter einem verminderten Druck zur Entfernung des organischen Lösungsmittels, um eine Nanopartikellösung zu erhalten.

11. Verfahren nach Anspruch 10, wobei das organische Lösungsmittel in Schritt (1) eingesetzt wird, vorzugsweise indem das Elemene in dem organischen Lösungsmittel gelöst und anschließend mit dem Injektionsöl vermischt wird;

oder wobei das Verfahren weiter einen Schritt des Lyophilisierens der erhaltenen Proteinträger-Nanopartikellösung umfasst;
oder wobei in Schritt (2) ein Scher- oder ein Ultraschallverfahren zum Vermischen verwendet wird und in Schritt (3) ein Hochdruckhomogenisations- oder ein Mikrostrahlhomogenisationsverfahren zur Homogenisierung verwendet wird.

12. Pharmazeutische Zusammensetzung nach einem der Ansprüche 1-9 zur Verwendung bei der Prophylaxe oder Behandlung von Krebs;
vorzugsweise ist der Krebs adrenokortikales Karzinom, kryptogene myeloide Metaplasie, AIDS-assoziierter Krebs, Analkarzinom, Appendixkarzinom, Astrozytom, Basalzellkarzinom, Cholangiokarzinom, Blasenkarzinom, Knochentumor, Gliom, Ependymom, Oligodendrogliom, Meningeom, Kraniopharyngeom, Hämangioblastom, Medulloblastom, neuroektodermaler Tumor, Sehbahn- und hypothalamisches Gliom, Glioblastom, Mammakarzinom, Bronchialadenom, Karzinoidtumor, ZNS-Lymphom, Zervixkarzinom, Colonkarzinom, Kolorektalkarzinom, chronische myeloproliferative Erkrankung, Endometriumkarzinom, Ependymom, Ösophaguskarzinom, Ewing-Tumorfamilie, Augentumor, Gallenblasenkarzinom, gastrointestinaler Karzinoidtumor, gastrointestinaler Stromatumor, Keimzelltumor, gestationeller Trophoblasttumor, Kopf-Hals-Tumor, Leberkarzinom, Larynxkarzinom, Leukämie, Lippen- und Mundhöhlenkarzinom, Lungenkarzinom, Lymphom, Medulloblastom, Melanom, Mesotheliom, metastasiertes Plattenepithelkarzinom des Halses, multiples endokrines Neoplasiesyndrom, myelodysplastisches Syndrom, myelodysplastische/myeloproliferative Erkrankung, Karzinom der Nasenhöhle und der Nasennebenhöhlen, Nasopharynxkarzinom, Neuroblastom, neuroendokriner Tumor, Oropharynxkarzinom, Hirntumor, Knochenmetastasierung, Magenkarzinom, Darmkarzinom, Ösophaguskarzinom, Ovarialkarzinom, Pankreaskarzinom, Mammakarzinom, Hautkarzinom, Parathyroidkarzinom, Peniskarzinom, Peritonealkarzinom, Pharynxkarzinom, Phäochromozytom, Pineoblastom und supratentorieller primitiver neuroektodermaler Tumor, Hypophysentumor, pleuropulmonales Blastom, Lymphom, primäres ZNS-Lymphom, pulmonale Lymphangiomyomatose, Rektumkarzinom, Nierenkarzinom, Nierenbecken- und Harnleiterkarzinom, Rhabdomyosarkom, Speicheldrüsenkarzinom, Hautkarzinom, Dünndarmkarzinom, Plattenepithelkarzinom, Hodentumor, Rachentumor, Thymom und thymisches Karzinom, Schilddrüsenkarzinom, Harnröhrenkarzinom oder Vaginalkarzinom.

13. Pharmazeutische Formulierung, die die pharmazeutische Zusammensetzung nach einem der Ansprüche 1-9 umfasst.

14. Pharmazeutische Formulierung nach Anspruch 13, wobei die pharmazeutische Formulierung eine feste Formulierung, eine flüssige Formulierung oder eine gasförmige Formulierung ist;
vorzugsweise ist die flüssige Formulierung eine stabile wässrige Suspension, die aus einem sterilen lyophilisierten Pulver rekonstituiert wird.

15. Pharmazeutische Formulierung nach Anspruch 13,

weiter umfassend andere Arzneimittel, einschließlich Antikrebsmittel;
vorzugsweise umfasst das Antikrebsmittel Temozolomid, Paclitaxel, Docetaxel, Taxane, Gemcitabin, ein Anti-VEGF-Arzneimittel oder ein PD-1-Antikörperarzneimittel.

16. Versiegelter Behälter, der die pharmazeutische Zusammensetzung nach einem der Ansprüche 1-9 oder die pharmazeutische Formulierung nach einem der Ansprüche 13-15 umfasst.

**Revendications**

1. Composition pharmaceutique comprenant de l'élémène, une huile pour injection et un vecteur protéique, dans laquelle l'huile pour injection est de l'huile de soja et le vecteur protéique est de l'albumine sérique humaine, et dans laquelle le rapport pondéral de l'huile de soja sur l'élémène est d'environ 0,35 à 3 et le rapport pondéral de l'albumine sérique humaine sur l'élémène est d'environ 0,5 à 20.

2. Composition pharmaceutique selon la revendication 1, dans laquelle la composition pharmaceutique se présente sous la forme de particules, et la taille de particule des particules est inférieure à 180 nm, préférablement de 50 à 180 nm, dans laquelle la taille de particule est déterminée sur la base d'un échantillon de 25 μL de la composition diluée avec 1 mL d'eau ultrapure et en utilisant un analyseur de taille de particule MALVERN ZETASIZER NANO ZS90, un angle de détection de 90° et une température de 25 °C.

3. Composition pharmaceutique selon la revendication 1, dans laquelle l'élémène est sélectionné parmi un ou plusieurs de α-élémène, β-élémène, γ-élémène et δ-élémène ; préférablement, l'élémène est β-élémène.

4. Composition pharmaceutique selon l'une quelconque des revendications 1 à 3, comprenant en outre un lyoprotecteur ; préférablement, le lyoprotecteur est sélectionné parmi un ou plusieurs du glucose, du sucrose, du maltose, du lactose, du mannose, de la tréhalose, de la glycine et du dextrane.

5. Composition pharmaceutique selon l'une quelconque des revendications 1 à 4, comprenant en outre un ou plusieurs parmi un agent régulateur iso-osmotique, un anti-oxydant, un conservateur et un régulateur de pH ;

   préférablement dans laquelle l'agent régulateur iso-osmotique est un ou plusieurs parmi la glycérine, le sorbitol, le mannitol ou le glucose ;
   ou dans laquelle le régulateur de pH est un ou plusieurs parmi l'hydroxyde de sodium, le citrate de sodium, l'acide citrique, l'acide phosphorique, l'acide acétique ou l'acide chlorhydrique ;
   ou dans laquelle le conservateur est sélectionné parmi un ou plusieurs d'esters d'alkyle d'hydroxybenzène, d'acide benzoïque, de benzoate de sodium, d'acide sorbique, d'acétate de chlorhexidine, de bromure de benzalkonium ;
   ou dans laquelle l'anti-oxydant est sélectionné parmi un ou plusieurs de sulfite de sodium, de bisulfite de sodium, de métabisulfite de sodium, de thiosulfate de sodium, d'acide ascorbique, d'hydroxyanisole butylé, de 2,6-di-tert-hydroxytoluène butylé, et de vitamine E.

6. Composition pharmaceutique selon l'une quelconque des revendications 1 à 5, dans laquelle aucun autre solvant organique que l'huile pour injection n'est inclus pendant son processus de préparation.

7. Composition pharmaceutique selon la revendication 6, dans laquelle, dans la composition pharmaceutique, le rapport pondéral de l'huile de soja sur l'élémène est d'environ 0,5 à 3 ; et le rapport pondéral de l'albumine sérique humaine sur l'élémène est d'environ 0,5 à 2,5 ;

   ou dans laquelle, dans la composition pharmaceutique, le rapport pondéral de l'huile de soja sur l'élémène est d'environ 0,35 à 1,5, préférablement de 0,35 à 0,75 ; et le rapport pondéral de l'albumine sérique humaine sur l'élémène est d'environ 1,5 à 10 ;
   ou dans laquelle, dans la composition pharmaceutique, le rapport pondéral de l'huile de soja sur l'élémène est d'environ 0,5 ; et le rapport pondéral de l'albumine sérique humaine sur l'élémène est d'environ 1,5 ;
   ou dans laquelle, dans la composition pharmaceutique, le rapport pondéral de l'huile de soja sur l'élémène est d'environ 0,75 ; et le rapport pondéral de l'albumine sérique humaine sur l'élémène est d'environ 5 ;
   ou dans laquelle, dans la composition pharmaceutique, le rapport pondéral de l'huile de soja sur l'élémène est d'environ 1,5 ; et le rapport pondéral de l'albumine sérique humaine sur l'élémène est d'environ 3 à 5.

8. Composition pharmaceutique selon l'une quelconque des revendications 1 à 5, dans laquelle un solvant organique est inclus pendant son processus de préparation ;
   préférablement dans laquelle le solvant organique est sélectionné parmi un ou plusieurs du chloroforme, du dichlorométhane, du butanol tertiaire, de l'isopropanol, de l'acétate d'éthyle, de l'éthanol, du tétrahydrofuranne, du dioxane, de l'acétonitrile, de l'acétone, du sulfoxyde de diméthyle, du formamide de diméthyle et du méthylpyrrolidone.

**9.** Composition pharmaceutique selon la revendication 8, dans laquelle la composition pharmaceutique se présente sous la forme de particules, et la taille de particule des particules est d'environ 70 à 150 nm, dans laquelle la taille de particule est déterminée sur la base d'un échantillon de 25 μL de la composition diluée avec 1 mL d'eau ultrapure et en utilisant un analyseur de taille de particule MALVERN ZETASIZER NANO ZS90, un angle de détection de 90° et une température de 25 °C ;
préférablement, dans la composition pharmaceutique, le rapport pondéral de l'huile de soja sur l'élémène est d'environ 2,4 ; et le rapport pondéral de l'albumine sérique humaine sur l'élémène est d'environ 3.

**10.** Procédé de préparation de la composition pharmaceutique selon l'une quelconque des revendications 1 à 9, comprenant les étapes consistant à :

(1) mélanger de l'élémène et une huile pour injection de façon homogène, ajouter facultativement un solvant organique, afin d'obtenir une solution de l'élémène et de l'huile pour injection ; dissoudre un vecteur protéique dans de l'eau afin d'obtenir une solution de vecteur protéique ;
(2) mélanger les deux solutions obtenues à l'étape (1) pour former une émulsion ;
(3) homogénéiser l'émulsion de l'étape (2) sous une pression élevée, et, facultativement, faire s'évaporer l'émulsion sous une pression réduite afin d'éliminer le solvant organique, afin d'obtenir une solution de nanoparticules.

**11.** Procédé selon la revendication 10, dans lequel le solvant organique est utilisé à l'étape (1), dissolvant préférablement l'élémène dans le solvant organique puis le mélangeant à l'huile pour injection ;

ou dans lequel le procédé comprend en outre une étape consistant à lyophiliser la solution de nanoparticules de vecteur protéique obtenue ;
ou dans lequel, à l'étape (2), un procédé de cisaillement ou à ultrasons est utilisé pour le mélange ; et à l'étape (3), un procédé d'homogénéisation haute-pression ou d'homogénéisation à micro-jet est utilisé pour l'homogénéisation.

**12.** Composition pharmaceutique selon l'une quelconque des revendications 1 à 9 pour utilisation dans la prévention ou le traitement du cancer ;
préférablement, le cancer est un carcinome corticosurrénalien, une métaplasie myéloïde cryptogénique, un cancer lié au SIDA, un cancer anal, un cancer appendiculaire, un astrocytome, un carcinome à cellules basales, un cholangiocarcinome, un cancer de la vessie, un cancer des os, un gliome, un épendymome, un oligodendrogliome, un méningiome, un craniopharyngiome, un hémangioblastome, un médulloblastome, une tumeur neuroectodermique, un glioblastome et un gliome hypothalamique et de la voie visuelle, un cancer du sein, un adénome bronchique, une tumeur carcinoïde, un lymphome du système nerveux central, un cancer du col de l'utérus, un cancer du côlon, un cancer colorectal, un trouble myéloprolifératif chronique, un cancer de l'endomètre, un épendymome, un cancer de l'œsophage, la famille des tumeurs d'Ewing, un cancer de l'œil, un cancer de la vésicule biliaire, une tumeur carcinoïde gastrointestinale, une tumeur stromale gastrointestinale, une tumeur à cellules germinales, une tumeur trophoblastique gestationnelle, un cancer de la tête et du cou, un cancer du foie, un cancer du larynx, une leucémie, cancer des lèvres et de la bouche, un cancer du poumon, un lymphome, un médulloblastome, un mélanome, un mésothéliome, un carcinome épidermoïque du cou métastatique, un syndrome de néoplasie endocrinienne multiple, un syndrome myélodysplastique, une maladie myélodysplastique/myéloproliférative, un carcinome de la cavité nasale et des sinus, un cancer du nasopharynx, un neuroblastome, un cancer neuroendocrinien, un cancer oropharyngé, une tumeur cérébrale, un cancer à métastases osseuse, un cancer gastrique, un cancer intestinal, un cancer de l'œsophage, un cancer de l'ovaire, un cancer du pancréas, un cancer du sein, un cancer de la peau, un carcinome de la parathyroïde, un carcinome du pénis, un cancer du péritoine, un carcinome du pharynx, un phéochromocytome, un pinéoblastome et tumeur neuroectodermique primitive supratentoriale, une tumeur pituitaire, un blastome pleuropulmonaire, un lymphome, un lymphome du système nerveux central primaire, une lymphangiomyomatose pulmonaire, un cancer rectal, un cancer du rein, un cancer de l'uretère et du pelvis rénal, un rhabdomyosarcome, un cancer des glandes salivaires, un cancer de la peau, un cancer de l'intestin grêle, un carcinome à cellules épithéliales squameuses, un cancer des testicules, un cancer de la gorge, un thymome et un carcinome thymique, un cancer de la thyroïde, un cancer de l'urètre ou un cancer du vagin.

**13.** Formulation pharmaceutique, comprenant la composition pharmaceutique selon l'une quelconque des revendications 1 à 9.

**14.** Formulation pharmaceutique selon la revendication 13, dans laquelle la formulation pharmaceutique est une formulation solide, une formulation liquide ou une formulation gazeuse ;

préférablement, la formulation liquide est une suspension aqueuse stable reconstituée à partir d'une poudre lyophilisée stérile.

**15.** Formulation pharmaceutique selon la revendication 13,

comprenant en outre d'autres médicaments, incluant des médicaments anticancéreux ;

préférablement, le médicament anticancéreux comprend du témozolomide, du paclitaxel, du docétaxel, du taxane, de la gemcitabine, un médicament anti-VEGF ou un médicament anticorps PD-1.

**16.** Récipient hermétique comprenant la composition pharmaceutique selon l'une quelconque des revendications 1 à 9 ou la formulation pharmaceutique selon l'une quelconque des revendications 13 à 15.

Figure 1

Figure 2

Figure 3

Figure 4

Figure 5

Figure 6

Figure 7

Ailineng NPs

Figure 8

Survival rate of U87R1 model

— Control group
--- TMZ 50mg/kg
···· ELE Mic 50mg/kg
-·-· Alb 50mg/kg

Figure 9

**EP 4 070 786 B1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2005186230 A